# EUROPEAN PATENT APPLICATION

(11) **EP 2 392 346 A1**
(43) Date of publication of application: **07.12.2011**
(21) Application number: 11172774.9
(22) Date of filing: 05.04.2007
(51) Int. Cl.: A61K 39/09

(54) **Streptococcus pneumoniae vaccine**

(30) Priority: 07.04.2006 GB 0607088
(62) Divisional of application: 07727880.2
(71) Applicant: GlaxoSmithKline Biologicals SA, 1330 Rixensart (BE)
(72) Inventor: Biemans, Ralph Leon, 1330 Rixensart (BE); Denoel, Philippe, 1330 Rixensart (BE); Poolman, Jan, 1330 Rixensart (BE); Prieels, Jean Paul, 1330 Rixensart (BE)
(74) Representative: Lubienski, Michael John

(57) **Abstract**

The present invention is in the field of pneumococcal capsular saccharide conjugate vaccines. Specifically, a multivalent *Streptococcus pneumoniae* immunogenic composition is provided with various (for instance 9 or more) conjugated capsular saccharides from different *S. pneumoniae* serotypes, wherein the composition comprises conjugated capsular saccharide 18C which is less than 80, 70, 60, 50, 40, 30, 20, 15 or 10 % O-Acetylated.

## Description

### Field of the Invention

The present invention relates to an improved *Streptococcus pneumonia* vaccine.

### Background of the Invention

Children less than 2 years of age do not mount an immune response to most polysaccharide vaccines, so it has been necessary to render the polysaccharides immunogenic by chemical conjugation to a protein carrier. Coupling the polysaccharide, a T-independent antigen, to a protein, a T-dependent antigen, confers upon the polysaccharide the properties of T dependency including isotype switching, affinity maturation, and memory induction.

However, there can be issues with repeat administration of polysaccharide-protein conjugates, or the combination of polysaccharide-protein conjugates to form multivalent vaccines. For example, it has been reported that a *Haemophilus influenzae* type b polysaccharide (PRP) vaccine using tetanus toxoid (TT) as the protein carrier was tested in a dosage-range with simultaneous immunization with (free) TT and a pneumococcal polysaccharide-TT conjugate vaccine following a standard infant schedule. As the dosage of the pneumococcal vaccine was increased, the immune response to the PRP polysaccharide portion of the Hib conjugate vaccine was decreased, indicating immune interference of the polysaccharide, possibly via the use of the same carrier protein (Dagan et al., Infect Immun. (1998); 66: 2093-2098)

The effect of the carrier-protein dosage on the humoral response to the protein itself has also proven to be multifaceted. In human infants it was reported that increasing the dosage of a tetravalent tetanus toxoid conjugate resulted in a decreased response to the tetanus carrier (Dagan *et al.* supra). Classical analysis of these effects of combination vaccines have been described as carrier induced epitopic suppression, which is not fully understood, but believed to result from an excess amount of carrier protein (Fattom, Vaccine 17: 126 (1999)). This appears to result in competition for Th-cells, by the B-cells to the carrier protein, and B-cells to the polysaccharide. If the B-cells to the carrier protein predominate, there are not enough Th-cells available to provide the necessary help for the B-cells specific to the polysaccharide. However, the observed immunological effects have been inconsistent, with the total amount of carrier protein in some instances increasing the immune response, and in other cases diminishing the immune response.

Hence there remain technical difficulties in combining multiple polysaccharide conjugates into a single, efficacious, vaccine formulation.

*Streptococcus pneumoniae* is a Gram-positive bacterium responsible for considerable morbidity and mortality (particularly in the young and aged), causing invasive diseases such as pneumonia, bacteraemia and meningitis, and diseases associated with colonisation, such as acute Otitis media. The rate of pneumococcal pneumonia in the US for persons over 60 years of age is estimated to be 3 to 8 per 100,000. In 20% of cases this leads to bacteraemia, and other manifestations such as meningitis, with a mortality rate close to 30% even with antibiotic treatment.

Pneumococcus is encapsulated with a chemically linked polysaccharide which confers serotype specificity. There are 90 known serotypes of pneumococci, and the capsule is the principle virulence determinant for pneumococci, as the capsule not only protects the inner surface of the bacteria from complement, but is itself poorly immunogenic. Polysaccharides are T-independent antigens, and can not be processed or presented on MHC molecules to interact with T-cells. They can however, stimulate the immune system through an alternate mechanism which involves cross-linking of surface receptors on B cells.

It was shown in several experiments that protection against invasive pneumococci disease is correlated most strongly with antibody specific for the capsule, and the protection is serotype specific.

*Streptococcus pneumoniae* is the most common cause of invasive bacterial disease and Otitis media in infants and young children. Likewise, the elderly mount poor responses to pneumococcal vaccines [Roghmann et al., (1987), J. Gerontol. 42:265-270], hence the increased incidence of bacterial pneumonia in this population [Verghese and Berk, (1983) Medicine (Baltimore) 62:271-285].

The major clinical syndromes caused by *S. pneumoniae* are widely recognized and discussed in all standard medical textbooks (Fedson DS, Muscher DM. In: Plotkin SA, Orenstein WA, editors. Vaccines. 4rth edition. PhiladelphiaWB Saunders Co, 2004a: 529-588). For instance, Invasive pneumococcal disease (IPD) is defined as any infection in which *S. pneumoniae* is isolated from the blood or another normally sterile site (Musher DM. Streptococcus pneumoniae. In Mandell GL, Bennett JE, Dolin R (eds). Principles and Practice of Infectious diseases (5th ed). New York, Churchill Livingstone, 2001, p2128-2147). Chronic obstructive pulmonary disease (COPD) is recognised as encompassing several conditions (airflow obstruction, chronic bronchitis, bronchiolitis or small airways disease and emphysema) that often coexist. Patients suffer exacerbations of their condition that are usually associated with increased breathlessness, and often have increased cough that may be productive of mucus or purulent sputum (Wilson, Eur Respir J 2001 17:995-1007). COPD is defined physiologically by the presence of irreversible or partially reversible airway obstruction in patients with chronic bronchitis and/or emphysema (Standards for the diagnosis and care of patients with chronic obstructive pulmonary disease. American Thoracic Society. Am J Respir Crit Care Med. 1995 Nov;152(5 Pt 2):S77-121). Exacerbations of COPD are often caused by bacterial (e.g. pneumococcal) infection (Sethi S, Murphy TF. Bacterial infection in chronic obstructive pulmonary disease in 2000: a state-of-the-art review. Clin Microbiol Rev. 2001 Apr;14(2):336-63).

Although there is a marketed vaccine Prevnar comprising saccharides from serotypes 4, 6B, 9V, 14, 18C, 19F and 23F all conjugated to carrier protein CRM197 (a non toxic variant of diphtheria toxin, little is know about the method of manufacture of this vaccine or the characteristics of its saccharides.

O- or N-acetylation is known to be important substituents in the formation of protective epitopes for various antigenic saccharides. It is generally not predictable whether the presence of acetyl groups is beneficial or detrimental to the epitope.

It is thus an object of the present invention to develop an improved formulation of a multiple serotype *Streptococcus pneumoniae* polysaccharide conjugate vaccine, in particular it is an object of the present invention to develop a vaccine with beneficial acetylation characteristics. In particular the present inventors have surprisingly found that de-O-acetylation of the capsular saccharide of serotype 18C may be beneficial in focusing the immune response on backbone epitopes which may be beneficial in raising an immune response protective against both 18C strains that are highly O-acetylated and those that are poorly O-acetylated.

### Brief description of Figures

**Figure 1****:** ELISA inhibition of pooled sera from mice immunized with SP1008 or Prevnar
**Figure 2****:** ELISA inhibition of pooled sera from mice immunized with SP1008 or experimental formulations containing PS18-DT_{AH} or PS18-TT_{AH} conjugates
**Figure 3****:** Opsonophagocytosis inhibition of pooled sera from mice immunized with SP1008 or experimental formulations containing PS18-DT_{AH} or PS18-TT_{AH} conjugates
**Figure 4****:** ELISA inhibition of pooled sera from guinea pig immunized with SP1008 or Prevnar
**Figure 5****:** ELISA inhibition of pooled sera from guinea pig immunized with SP1008 or experimental formulations containing PS18-DT_{AH} or PS18-TT_{AH} conjugates
**Figure 6****:** Opsonophagocytosis inhibition of pooled sera from guinea pig immunized with SP1008 or experimental formulations containing PS18-DT_{AH} or PS18-TT_{AH} conjugates
**Figure 7****:** Inhibition of the pooled sera from 11 PnPD&Di007 infants immunized with SP1008 or Prevnar

### Description of the Invention

Thus in one embodiment of the invention there is provided a *Streptococcus pneumoniae* immunogenic composition comprising 9 or more, 10 or more, 11 or more, or 13 or more capsular saccharides from different *S. pneumoniae* serotypes which are conjugated to a carrier protein, wherein the composition comprises conjugated capsular saccharide 18C which is less than 80, 70, 60, 50, 40, 30, 20, 15 or 10 % O-Acetylated.

By % O- or N-acetylation it is meant the % of acetylation of a given saccharide sample relative to 100% (where each repeat unit is fully acetylated relative to its acetylated structure). For ease of reference, the structure of various saccharide repeat units are given below.

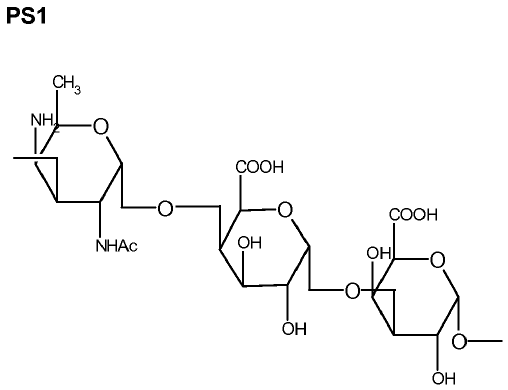

PS1 is known to be O-acetylated either on the first or second -OH group of the middle sugar of the repeat unit.

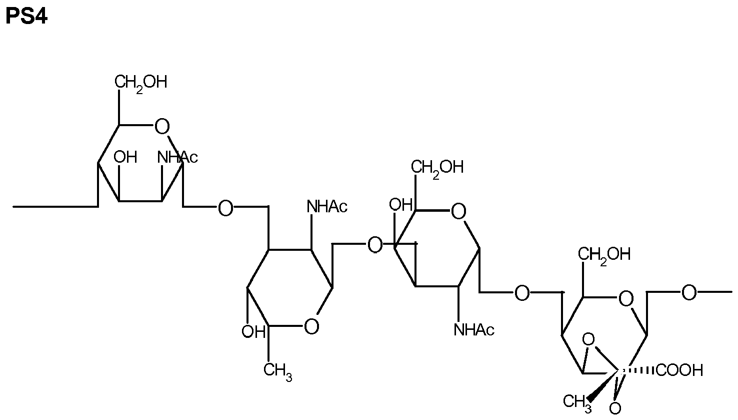

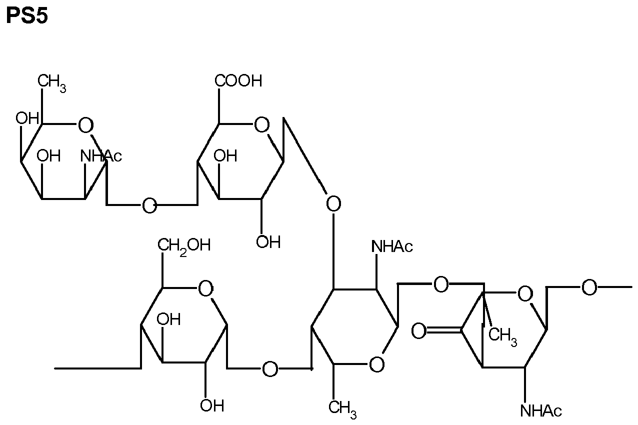

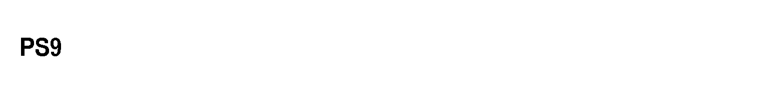

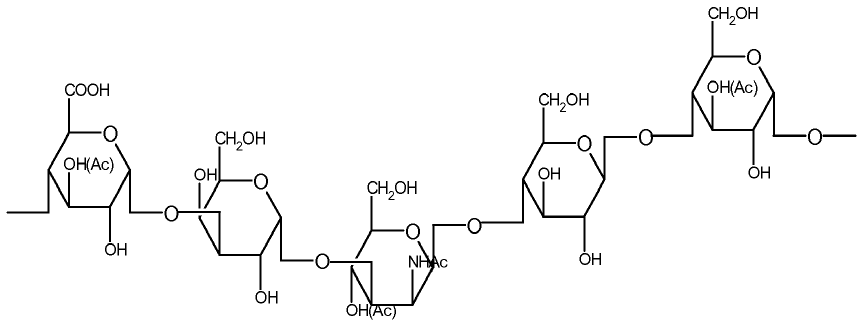

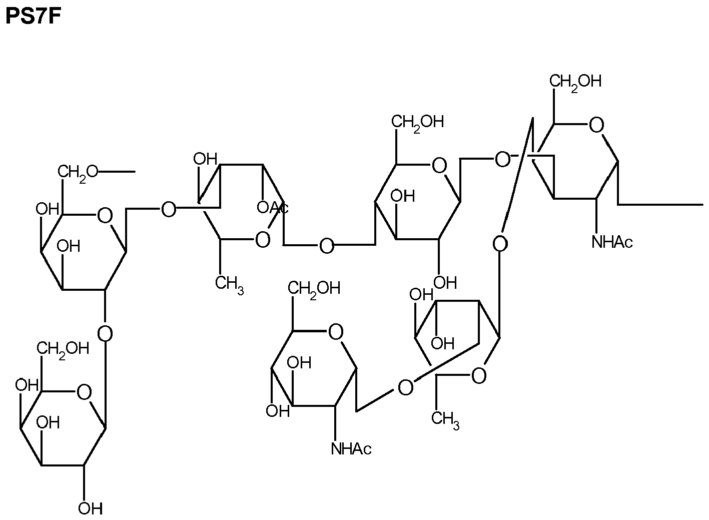

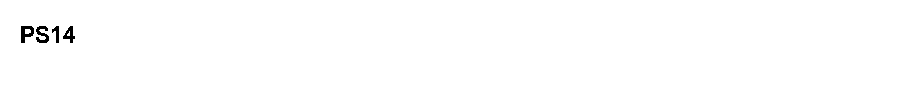

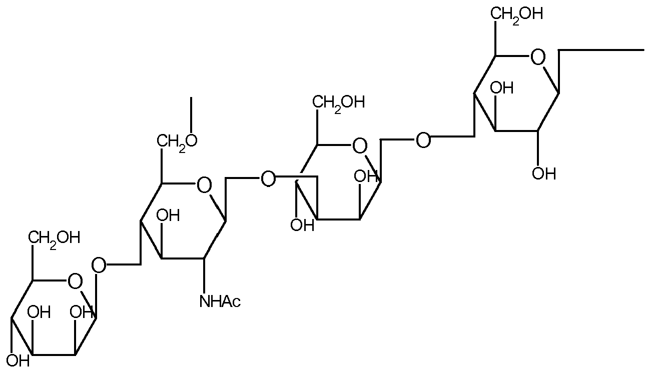

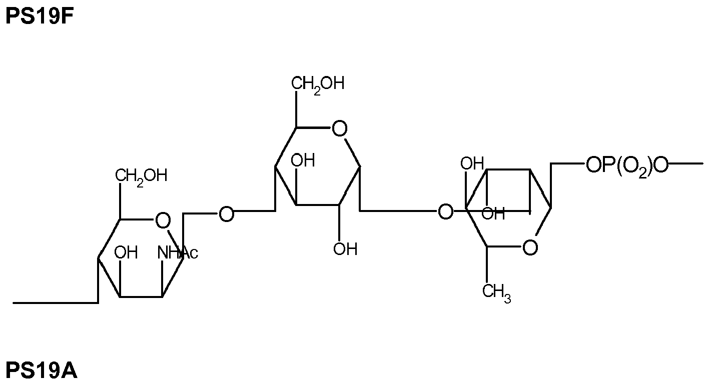

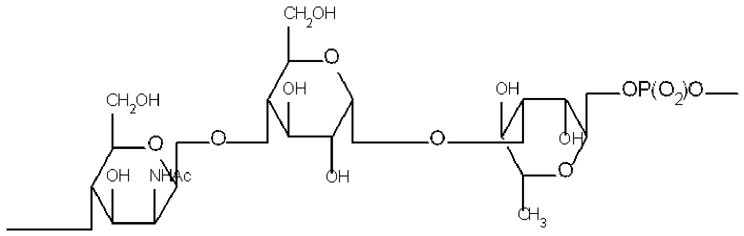

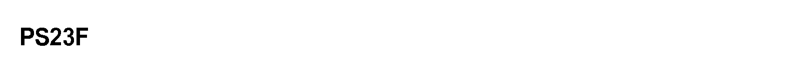

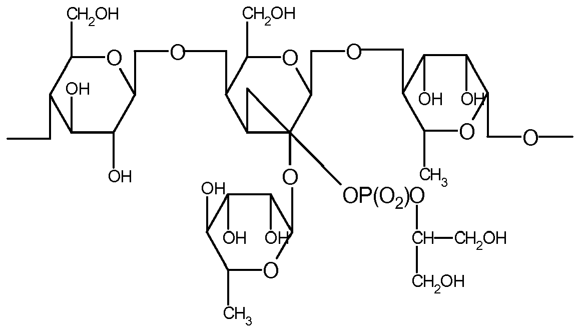

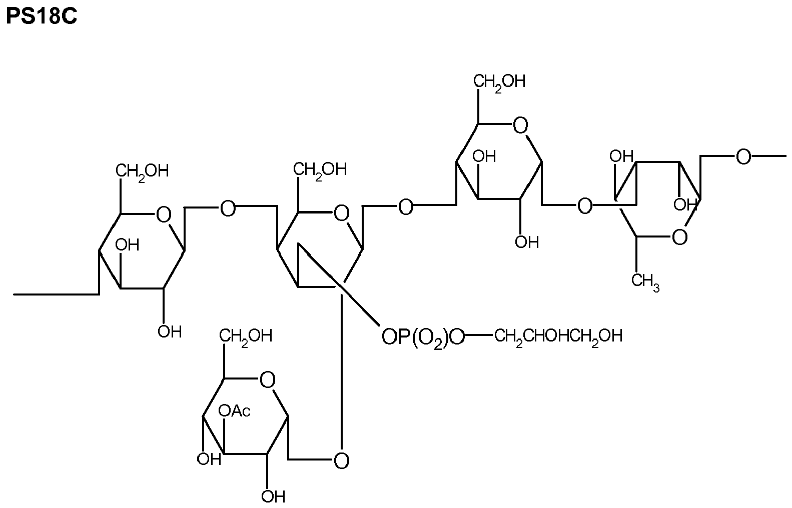

Saccharides can be de-O-acetylated by various means known in the art. Measurement of acetylation is possible by various methods known in the art; for instance NMR may be used. In one aspect the capsular saccharide 18C is de-O-acetylated through treatment with acid before its conjugation. For instance treatment with 1 M acetic acid for 40 hours at 60 °C (see WO 96/05859 example 4) yields 18C saccharide that is approximately 17% O-acetylated by NMR. A similar treatment at 100 °C for 5-6 hours yields 30-50% O-acetylated 18C. Native 18C from an acetylated 18C strain tends to be highly O-acetylated (>90%).

In one embodiment the 18C saccharide of the invention is conjugated to tetanus toxoid (TT), optionally wherein 18C is the only *S. pneumoniae* capsular saccharide conjugated to TT. The capsular saccharides from different *S. pneumoniae* serotypes may be conjugated to 2 or more different carrier proteins. In one embodiment 18C is not conjugated to pneumolysin. In another embodiment 18C is not conjugated to CRM197. 18C capsular saccharide of the invention may be conjugated by many known means (see below). In one embodiment it is not conjugated with reductive amination chemistry, in a further embodiment it is conjugated with reductive amination chemistry (see below).

Typically the *Streptococcus pneumoniae* immunogenic composition (or vaccine) of the present invention will comprise capsular saccharide antigens (preferably conjugated), wherein the saccharides are derived from at least 9 or at least ten serotypes of *S. pneumoniae.* The number of S. *pneumoniae* capsular saccharides can range from 9 or 10 different serotypes (or "V", valences) to 23 different serotypes (23V). In one embodiment there are 9, 10, 11, 13 or 15 different serotypes. In another embodiment of the invention, the vaccine may comprise conjugated *S. pneumoniae* saccharides and unconjugated *S. pneumoniae* saccharides. Preferably, the total number of saccharide serotypes is less than or equal to 23. For example, the invention may comprise 10 conjugated serotypes and 13 unconjugated saccharides. In a similar manner, the vaccine may comprise 13 or 16 conjugated saccharides and 10, or 7 respectively, unconjugated saccharides.

In one embodiment the multivalent pneumococcal vaccine of the invention will be selected from the following serotypes 1, 2, 3, 4, 5, 6A, 6B, 7F, 8, 9N, 9V, 10A, 11A, 12F, 14, 15B, 17F, 18C, 19A, 19F, 20, 22F, 23F and 33F, although it is appreciated that one or two other serotypes could be substituted depending on the age of the recipient receiving the vaccine and the geographical location where the vaccine will be administered. For example, a 10-valent vaccine may comprise polysaccharides from serotypes 1, 4, 5, 6B, 7F, 9V, 14, 18C, 19F and 23F. An 11-valent vaccine may also include capsular saccharide from serotype 3. A 12 or 13-valent paediatric (infant) vaccine may also include serotypes 6A and 19A, or 6A and 22F, or 19A and 22F, or 6A and 15B, or 19A and 15B, or 22F and 15B (with the 10 or 11 valent formulation, respectively), whereas a 13-valent elderly vaccine may include serotypes 8 and 12F, or 8 and 15B, or 8 and 19A, or 8 and 22F, or 12F and 15B, or 12F and 19A, or 12F and 22F, or 15B and 19A, or 15B and 22F (with the 11 valent formulation). A 14 valent paediatric vaccine may include the 10 valent formulation described above supplemented with serotypes 3, 6A, 19A and 22F; serotypes 6A, 8, 19A and 22F; serotypes 6A, 12F, 19A and 22F; serotypes 6A, 15B, 19A and 22F; serotypes 3, 8, 19A and 22F; serotypes 3, 12F, 19A and 22F; serotypes 3, 15B, 19A and 22F; serotypes 3, 6A, 8 and 22F; serotypes 3, 6A, 12F and 22F; or serotypes 3, 6A, 15B and 22F.

The composition in one embodiment includes capsular saccharides derived from serotypes 1, 4, 5, 6B, 7F, 9V, 14, 18C, 19F and 23F (preferably conjugated). In a further embodiment of the invention at least 11 saccharide antigens (preferably conjugated) are included, for example capsular saccharides derived from serotypes 1, 3, 4, 5, 6B, 7F, 9V, 14, 18C, 19F and 23F. In a further embodiment of the invention, at least 12 or 13 saccharide antigens are included, for example a vaccine may comprise capsular saccharides derived from serotypes 1, 3, 4, 5, 6A, 6B, 7F, 9V, 14, 18C, 19A, 19F and 23F or capsular saccharides derived from serotypes 1, 3, 4, 5, 6B, 7F, 9V, 14, 18C, 19A, 19F, 22F and 23F, although further saccharide antigens, for example 23 valent (such as serotypes 1, 2, 3, 4, 5, 6B, 7F, 8, 9N, 9V, 10A, 11A, 12F, 14, 15B, 17F, 18C, 19A, 19F, 20, 22F, 23F and 33F), are also contemplated by the invention.

Where capsular saccharide from serotype 4 is present in one embodiment it is more than 50, 60, 70, 80, 90 % N-Acetylated. Where capsular saccharide from serotype 9V is present in one embodiment it is more than 50, 60, 70, 80, 90 % N-Acetylated and/or more than 50, 60, 70, 80, 90 % O-Acetylated. Where capsular saccharide from serotype 14 is present in one embodiment it is more than 50, 60, 70, 80, 90 % N-Acetylated. Where capsular saccharide from serotype 1 is present in one embodiment it is more than 50, 60, 70, 80, 90 % N-Acetylated and/or more than 50, 60, 70, 80, 90 % O-Acetylated.. Where capsular saccharide from serotype 5 is present in one embodiment it is more than 50, 60, 70, 80, 90 % N-Acetylated. Where capsular saccharide from serotype 7F is present in one embodiment it is more than 50, 60, 70, 80, 90 % N-Acetylated and/or more than 50, 60, 70, 80, 90 % O-Acetylated. Where capsular saccharide from serotype 4 is present in one embodiment it is Where capsular saccharide from serotype 4 is present in one embodiment it is

The vaccine of the present invention may comprise protein D (PD) from *Haemophilus influenzae* (see e.g. EP 0594610) - in particular from non-typeable *Haemophilus influenzae* (ntHi). ntHi is a key causative organism of otitis media, and the present inventors have shown that including this protein in a *Streptococcus pneumoniae* vaccine will provide a level of protection against *Haemophilus influenzae* related otitis media (Prymula et al. 2006 The Lancet 367:740-748). In one embodiment, the vaccine composition comprises protein D. In one aspect, PD is present as a carrier protein for one or more of the saccharides. In another aspect, protein D could be present in the vaccine composition as a free protein. In a further aspect, protein D is present both as a carrier protein and as free protein. Protein D may be used as a full length protein or as a fragment (WO0056360). In a further aspect, protein D is present as a carrier protein for the majority of the saccharides, for example 6, 7, 8, 9 or more of the saccharides may be conjugated to protein D. In this aspect, protein D may also be present as free protein.

The vaccine of the present invention may comprise two or more different types of carrier protein. Each type of carrier protein may act as carrier for more than one saccharide, which saccharides may be the same or different. Saccharides may be conjugated to the same carrier in the same reaction or may be individually conjugated to the same carrier protein. For example, serotypes 3 and 4 may be conjugated to the same carrier protein, either to the same molecule of carrier protein or to different molecules of the same carrier protein. In one embodiment, two or more different saccharides may be conjugated to the same carrier protein, either to the same molecule of carrier protein or to different molecules of the same carrier protein.

Each/any *Streptococcus pneumoniae* capsular saccharide may be conjugated to a carrier protein independently selected from the group consisting of TT, DT, CRM197, fragment C of TT, PhtD, PhtDE fusions (particularly those described in WO 01/98334 and WO 03/54007), detoxified pneumolysin and protein D. In one aspect capsular saccharide from serotype 19F may be conjugated to DT or CRM 197, preferably DT. A more complete list of protein carriers that may be used in the conjugates of the invention is presented below

The carrier protein conjugated to one or more of the *S. pneumoniae* capsular saccharides in the conjugates present in the immunogenic compositions of the invention is optionally a member of the polyhistidine triad family (Pht) proteins, fragments or fusion proteins thereof. The PhtA, PhtB, PhtD or PhtE proteins may have an amino acid sequence sharing 80%, 85%, 90%, 95%, 98%, 99% or 100% identity with a sequence disclosed in WO 00/37105 or WO 00/39299 (e.g. with amino acid sequence 1-838 or 21-838 of SEQ ID NO: 4 of WO 00/37105 for PhtD). For example, fusion proteins are composed of full length or fragments of 2, 3 or 4 of PhtA, PhtB, PhtD, PhtE. Examples of fusion proteins are PhtA/B, PhtA/D, PhtA/E, PhtB/A, PhtB/D, PhtB/E. PhtD/A. PhtD/B, PhtD/E, PhtE/A, PhtE/B and PhtE/D, wherein the proteins are linked with the first mentioned at the N-terminus (see for example WO01/9833

Where fragments of Pht proteins are used (separately or as part of a fusion protein), each fragment optionally contains one or more histidine triad motif(s) and/or coiled coil regions of such polypeptides. A histidine triad motif is the portion of polypeptide that has the sequence HxxHxH where H is histidine and x is an amino acid other than histidine. A coiled coil region is a region predicted by "Coils" algorithm Lupus, A et al (1991) Science 252; 1162-1164. In an embodiment the or each fragment includes one or more histidine triad motif as well as at least one coiled coil region. In an embodiment, the or each fragment contains exactly or at least 2, 3, 4 or 5 histidine triad motifs (optionally, with native Pht sequence between the 2 or more triads, or intra-triad sequence that is more than 50, 60, 70, 80, 90 or 100 % identical to a native pneumococcal intra-triad Pht sequence - e.g. the intra-triad sequence shown in SEQ ID NO: 4 of WO 00/37105 for PhtD). In an embodiment, the or each fragment contains exactly or at least 2, 3 or 4 coiled coil regions. In an embodiment a Pht protein disclosed herein includes the full length protein with the signal sequence attached, the mature full length protein with the signal peptide (for example 20 amino acids at N-terminus) removed, naturally occurring variants of Pht protein and immunogenic fragments of Pht protein (e.g. fragments as described above or polypeptides comprising at least 15 or 20 contiguous amino acids from an amino acid sequence in WO00/37105 or WO00/39299 wherein said polypeptide is capable of eliciting an immune response specific for said amino acid sequence in WO00/37105 or WO00/39299).

In particular, the term "PhtD" as used herein includes the full length protein with the signal sequence attached, the mature full length protein with the signal peptide (for example 20 amino acids at N-terminus) removed, naturally occurring variants of PhtD and immunogenic fragments of PhtD (e.g. fragments as described above or polypeptides comprising at least 15 or 20 contiguous amino acids from a PhtD amino acid sequence in WO00/37105 or WO00/39299 wherein said polypeptide is capable of eliciting an immune response specific for said PhtD amino acid sequence in WO00/37105 or WO00/39299 (e.g. SEQ ID NO: 4 of WO 00/37105 for PhtD).

If the protein carrier is the same for 2 or more saccharides in the composition, the saccharides could be conjugated to the same molecule of the protein carrier (carrier molecules having 2 more different saccharides conjugated to it) [see for instance WO 04/083251]. Alternatively the saccharides may each be separately conjugated to different molecules of the protein carrier (each molecule of protein carrier only having one type of saccharide conjugated to it).

Examples of carrier proteins which may be used in the present invention are DT (Diphtheria toxoid), TT (tetanus toxoid) or fragment C of TT, DT CRM197 (a DT mutant) other DT point mutants, such as CRM176, CRM228, CRM 45 (Uchida et al J. Biol. Chem. 218; 3838-3844, 1973); CRM 9, CRM 45, CRM102, CRM 103 and CRM107 and other mutations described by Nicholls and Youle in Genetically Engineered Toxins, Ed: Frankel, Maecel Dekker Inc, 1992; deletion or mutation of Glu-148 to Asp, Gln or Ser and/or Ala 158 to Gly and other mutations disclosed in US 4709017 or US 4950740; mutation of at least one or more residues Lys 516, Lys 526, Phe 530 and/or Lys 534 and other mutations disclosed in US 5917017 or US 6455673; or fragment disclosed in US 5843711, pneumococcal pneumolysin [Ply] (Kuo et al (1995) Infect Immun 63; 2706-13) including ply detoxified in some fashion for example chemically: dPLY-GMBS (WO 04081515, PCT/EP2005/010258) or dPLY-formol; or genetically mutated to be less toxic (using for instance mutations known in the art), PhtX, including PhtA, PhtB, PhtD, PhtE and fusions of Pht proteins for example PhtDE fusions, PhtBE fusions, (WO 01/98334 and WO 03/54007) (Pht A-E are described in more detail below) OMPC (meningococcal outer membrane protein - usually extracted from *N. meningitidis* serogroup B - EP0372501), PorB (from *N. meningitidis*), PD (*Haemophilus influenzae* protein D - see, e.g., EP 0 594 610 B), or immunologically functional equivalents thereof, synthetic peptides (EP0378881, EP0427347), heat shock proteins (WO 93/17712, WO 94/03208), pertussis proteins (WO 98/58668, EP0471177), cytokines, lymphokines, growth factors or hormones (WO 91/01146), artificial proteins comprising multiple human CD4+ T cell epitopes from various pathogen derived antigens (Falugi et al (2001) Eur J Immunol 31; 3816-3824) such as N19 protein (Baraldoi et al (2004) Infect Immun 72; 4884-7) pneumococcal surface protein PspA (WO 02/091998), iron uptake proteins (WO 01/72337), toxin A or B of *C. difficile* (WO 00/61761).

Nurkka et al Pediatric Infectious Disease Journal. 23(11):1008-14, 2004 Nov*.* described an 11 valent pneumococcal vaccine with all serotypes conjugated to PD. However, the present inventors have shown that opsonophagocytic activity was improved for antibodies induced with conjugates having 19F conjugated to DT compared with 19F conjugated to PD. In addition, the present inventors have shown that a greater cross reactivity to 19A is seen with 19F conjugated to DT. It is therefore a feature of the composition of the present invention that serotype 19F is conjugated to DT or CRM 197. In one aspect, serotype 19F is conjugated to DT. The remaining saccharide serotypes of the immunogenic composition may all be conjugated to one or more carrier proteins that are not DT (i.e. only 19F is conjugated to DT), or may be split between one or more carrier proteins that are not DT or CRM197 and DT or CRM197 itself. In one embodiment, 19F is conjugated to DT or CRM 197 and all of the remaining serotypes are conjugated to PD. In a further embodiment, 19F is conjugated to DT or CRM 197, and the remaining serotypes are split between PD, and TT or DT or CRM 197. In a further embodiment, 19F is conjugated to DT or CRM 197 and no more than one (or two or three) saccharide is conjugated to TT. In one aspect of this embodiment, said one (or two) saccharide is 18C and/or 12F. In a further embodiment, 19F is conjugated to DT or CRM 197 and no more than two saccharides are conjugated to TT. In a further embodiment, 19F is conjugated to DT or CRM 197, and the remaining serotypes are split between PD, TT and DT or CRM 197. In a further embodiment, 19F is conjugated to DT or CRM 197, and the remaining serotypes are split between PD, TT and pneumolysin. In a further embodiment, 19F is conjugated to DT or CRM 197, and the remaining serotypes are split between PD, TT and CRM 197. In a further embodiment, 19F is conjugated to DT or CRM197 and the remaining serotypes are split between PD, TT, pneumolysin and optionally PhtD. In a further aspect of the invention 18C capsular saccharide is conjugated to TT [optionally with one or two other saccharides conjugated to TT] and the remaining serotypes are split between PD, DT (or CRM197), pneumolysin and optionally PhtD.

In one embodiment, the immunogenic composition of the invention comprises protein D from *Haemophilus influenzae.* Within this embodiment, If PD is not one of the carrier proteins used to conjugate any saccharides, then PD may be present in the vaccine composition as free protein. If PD is one of the carrier proteins used to conjugate saccharides in the composition of the invention, then PD may optionally be present in the vaccine composition as free protein.

The term "saccharide" throughout this specification may indicate polysaccharide or oligosaccharide and includes both. Polysaccharides are isolated from bacteria and may be sized to some degree by known methods (see for example EP497524 and EP497525; Szu et al. - Carbohydrate Research Vol 152 p7-20 (1986)) and preferably by microfluidisation. Polysaccharides can be sized in order to reduce viscosity in polysaccharide samples and/or to improve filterability for conjugated products. Oligosaccharides have a low number of repeat units (typically 5-30 repeat units) and are typically hydrolysed polysaccharides

Capsular polysaccharides of *Streptococcus pneumoniae* comprise repeating oligosaccharide units which may contain up to 8 sugar residues. For a review of the oligosaccharide units for the key *Streptococcus pneumoniae* serotypes see JONES, Christopher. Vaccines based on the cell surface carbohydrates of pathogenic bacteria. An. Acad. Bras. Ciênc., June 2005, vol.77, no.2, p.293-324. ISSN 0001-3765. In one embodiment, a capsular saccharide antigen may be a native or full length polysaccharide (i.e. polysaccharide as prepared from *S. pneumoniae* without any sizing or hydrolysis steps), however in others it may be one or several repeat units (oligosaccharide), or a shorter than native length polysaccharide or oligosaccharide chain of repeating units. In one embodiment, all of the saccharides present in the vaccine are polysaccharides. Full length polysaccharides may be "sized" i.e. their size may be reduced by various methods such as acid hydrolysis treatment, hydrogen peroxide treatment, sizing by emulsiflex® followed by a hydrogen peroxide treatment to generate oligosaccharide fragments or microfluidization.

The inventors have also noted that the focus of the art has been to use oligosaccharides for ease of conjugate production. The inventors have found that by using native or slightly sized polysaccharide conjugates, one or more of the following advantages may be realised: 1) a conjugate having high immunogenicity which is filterable, 2) the ratio of polysaccharide to protein in the conjugate can be altered such that the ratio of polysaccharide to protein (w/w) in the conjugate may be increased (which can have an effect on the carrier suppression effect), 3) immunogenic conjugates prone to hydrolysis may be stabilised by the use of larger saccharides for conjugation. The use of larger polysaccharides can result in more cross-linking with the conjugate carrier and may lessen the liberation of free saccharide from the conjugate. The conjugate vaccines described in the prior art tend to depolymerise the polysaccharides prior to conjugation in order to improve conjugation. The present inventors have found that saccharide conjugate vaccines retaining a larger size of saccharide can provide a good immune response against pneumococcal disease.

The immunogenic composition of the invention may thus comprise one or more saccharide conjugates wherein the average size (e.g. weight-average molecular weight; Mw) of each saccharide before conjugation is above 80 x10³, 100 x10³, 200 x10³, 300 x10³, 400 x10³, 500 x10³ or 1000 x10³ (e.g. 80 x10³ to 1000 x10³; 90 x10³ to 500 x10³ ; 100 x10³ to 400 x10³; 200 x10³ to 300 x10³ ). In one embodiment one or more saccharide conjugates of the invention should have an average size of saccharide preconjugation of 50-1600, 80-1400, 100-1000, 150-500, or 200-400 kDa (note that where average size is M_{w}, 'kDa' units should be replaced herein with 'x10³'). In one embodiment the conjugate post conjugation should be readily filterable through a 0.2 micron filter such that a yield of more than 50, 60, 70, 80, 90 or 95% is obtained post filtration compared with the pre filtration sample.

For the purposes of the invention, "native polysaccharide" refers to a saccharide that has not been subjected to a process (e.g. post-purification), the purpose of which is to reduce the size of the saccharide. A polysaccharide can become slightly reduced in size during normal purification procedures. Such a saccharide is still native. Only if the polysaccharide has been subjected to sizing techniques would the polysaccharide not be considered native.

For the purposes of the invention, "sized by a factor up to x2" means that the saccharide is subject to a process intended to reduce the size of the saccharide but to retain a size more than half the size of the native polysaccharide. X3, x4 etc. are to be interpreted in the same way i.e. the saccharide is subject to a process intended to reduce the size of the polysaccharide but to retain a size more than a third, a quarter etc. the size of the native polysaccharide.

In an aspect of the invention, the immunogenic composition comprises *Streptococcus pneumoniae* saccharides from at least 9 or 10 serotypes conjugated to a carrier protein, wherein at least 1, 2, 3, 4, 5, 6, 7, 8, 9, 10 or each *S. pneumoniae* saccharide is native polysaccharide.

In an aspect of the invention, the immunogenic composition comprises *Streptococcus pneumoniae* saccharides from at least 9 or 10 serotypes conjugated to a carrier protein, wherein at least 1, 2, 3, 4, 5, 6, 7, 8, 9, 10 or each *S. pneumoniae* saccharide is sized by a factor up to x2, x3, x4, x5, x6, x7, x8, x9 or x10. In one embodiment of this aspect, the majority of the saccharides, for example 6, 7, 8 or more of the saccharides are sized by a factor up to x2, x3, x4, x5, x6, x7, x8, x9 or x 10.

The molecular weight or average molecular weight (or size) of a saccharide herein refers to the weight-average molecular weight (Mw) of the saccharide measured prior to conjugation and is measured by MALLS.

The MALLS technique is well known in the art (e.g. as described in example 2). For MALLS analysis of pneumococcal saccharides, two columns (TSKG6000 and 5000PWxl) may be used in combination and the saccharides are eluted in water. Saccharides are detected using a light scattering detector (for instance Wyatt Dawn DSP equipped with a 10mW argon laser at 488nm) and an inferometric refractometer (for instance Wyatt Otilab DSP equipped with a P100 cell and a red filter at 498nm).

In an embodiment the *S. pneumoniae* saccharides are native polysaccharides or native polysaccharides which have been reduced in size during a normal extraction process.

In an embodiment, the *S. pneumoniae* saccharides are sized by mechanical cleavage, for instance by microfluidisation or sonication. Microfluidisation and sonication have the advantage of decreasing the size of the larger native polysaccharides sufficiently to provide a filterable conjugate. Sizing is by a factor of no more than x20, x10, x8, x6, x5, x4, x3 or x2.

In an embodiment, the immunogenic composition comprises *S. pneumoniae* conjugates that are made from a mixture of native polysaccharides and saccharides that are sized by a factor of no more than x20. In one aspect of this embodiment, the majority of the saccharides, for example 6, 7, 8 or more of the saccharides are sized by a factor of up to x2, x3, x4, x5 or x6.

In an embodiment, the *Streptococcus pneumoniae* saccharide is conjugated to the carrier protein via a linker, for instance a bifunctional linker. The linker is optionally heterobifunctional or homobifunctional, having for example a reactive amino group and a reactive carboxylic acid group, 2 reactive amino groups or two reactive carboxylic acid groups. The linker has for example between 4 and 20, 4 and 12, 5 and 10 carbon atoms. A possible linker is ADH. Other linkers include B-propionamido (WO 00/10599), nitrophenyl-ethylamine (Gever et al (1979) Med. Microbiol. Immunol. 165; 171-288), haloalkyl halides (US4057685), glycosidic linkages (US4673574, US4808700), hexane diamine and 6-aminocaproic acid (US4459286). In an embodiment, ADH is used as a linker for conjugating saccharide from serotype 18C.

The saccharide conjugates present in the immunogenic compositions of the invention may be prepared by any known coupling technique. The conjugation method may rely on activation of the saccharide with 1-cyano-4-dimethylamino pyridinium tetrafluoroborate (CDAP) to form a cyanate ester. The activated saccharide may thus be coupled directly or via a spacer (linker) group to an amino group on the carrier protein. For example, the spacer could be cystamine or cysteamine to give a thiolated polysaccharide which could be coupled to the carrier via a thioether linkage obtained after reaction with a maleimide-activated carrier protein (for example using GMBS) or a haloacetylated carrier protein (for example using iodoacetimide [e.g. ethyl iodoacetimide HCl] or N-succinimidyl bromoacetate or SIAB, or SIA, or SBAP). Preferably, the cyanate ester (optionally made by CDAP chemistry) is coupled with hexane diamine or ADH and the amino-derivatised saccharide is conjugated to the carrier protein using carbodiimide (e.g. EDAC or EDC) chemistry via a carboxyl group on the protein carrier. Such conjugates are described in PCT published application WO 93/15760 Uniformed Services University and WO 95/08348 and WO 96/29094

Other suitable techniques use carbodiimides, hydrazides, active esters, norborane, p-nitrobenzoic acid, N-hydroxysuccinimide, S-NHS, EDC, TSTU. Many are described in WO 98/42721. Conjugation may involve a carbonyl linker which may be formed by reaction of a free hydroxyl group of the saccharide with CDI (Bethell et al J. Biol. Chem. 1979, 254; 2572-4, Hearn et al J. Chromatogr. 1981. 218; 509-18) followed by reaction of with a protein to form a carbamate linkage. This may involve reduction of the anomeric terminus to a primary hydroxyl group, optional protection/deprotection of the primary hydroxyl group' reaction of the primary hydroxyl group with CDI to form a CDI carbamate intermediate and coupling the CDI carbamate intermediate with an amino group on a protein.

The conjugates can also be prepared by direct reductive amination methods as described in US 4365170 (Jennings), US 4673574 (Anderson) and WO 96/05859. Other methods are described in EP-0-161-188, EP-208375 and EP-0-477508.

A further method involves the coupling of a cyanogen bromide (or CDAP) activated saccharide derivatised with adipic acid hydrazide (ADH) to the protein carrier by Carbodiimide condensation (Chu C. et al Infect. Immunity, 1983 245 256), for example using EDAC.

In an embodiment, a hydroxyl group (preferably an activated hydroxyl group for example a hydroxyl group activated to make a cyanate ester [e.g. with CDAP]) on a saccharide is linked to an amino or carboxylic group on a protein either directly or indirectly (through a linker). Where a linker is present, a hydroxyl group on a saccharide is preferably linked to an amino group on a linker, for example by using CDAP conjugation. A further amino group in the linker for example ADH) may be conjugated to a carboxylic acid group on a protein, for example by using carbodiimide chemistry, for example by using EDAC. In an embodiment, the pneumococcal capsular saccharide(s) is conjugated to the linker first before the linker is conjugated to the carrier protein. Alternatively the linker may be conjugated to the carrier before conjugation to the saccharide.

A combination of techniques may also be used, with some saccharide-protein conjugates being prepared by CDAP, and some by reductive amination.

In general the following types of chemical groups on a protein carrier can be used for coupling / conjugation:
A) Carboxyl (for instance via aspartic acid or glutamic acid). In one embodiment this group is linked to amino groups on saccharides directly or to an amino group on a linker with carbodiimide chemistry e.g. with EDAC.
B) Amino group (for instance via lysine). In one embodiment this group is linked to carboxyl groups on saccharides directly or to a carboxyl group on a linker with carbodiimide chemistry e.g. with EDAC. In another embodiment this group is linked to hydroxyl groups activated with CDAP or CNBr on saccharides directly or to such groups on a linker; to saccharides or linkers having an aldehyde group; to saccharides or linkers having a succinimide ester group.
C) Sulphydryl (for instance via cysteine). In one embodiment this group is linked to a bromo or chloro acetylated saccharide or linker with maleimide chemistry. In one embodiment this group is activated/modified with bis diazobenzidine.
D) Hydroxyl group (for instance via tyrosine). In one embodiment this group is activated/modified with bis diazobenzidine.
E) Imidazolyl group (for instance via histidine). In one embodiment this group is activated/modified with bis diazobenzidine.
F) Guanidyl group (for instance via arginine).
G) Indolyl group (for instance via tryptophan).

On a saccharide, in general the following groups can be used for a coupling: OH, COOH or NH2. Aldehyde groups can be generated after different treatments known in the art such as: periodate, acid hydrolysis, hydrogen peroxide, etc.

### Direct coupling approaches:

Saccharide-OH + CNBr or CDAP -----> cyanate ester + NH2-Prot ----> conjugate
Saccharide-aldehyde + NH2-Prot ----> Schiff base + NaCNBH3 ----> conjugate
Saccharide-COOH + NH2-Prot + EDAC ----> conjugate
Saccharide-NH2 + COOH-Prot + EDAC ----> conjugate

### Indirect coupling via spacer (linker) approaches:

Saccharide-OH + CNBr or CDAP ---> cyanate ester + NH2----NH2 ----> saccharideNH2 + COOH-Prot + EDAC -----> conjugate

Saccharide-OH + CNBr or CDAP ----> cyanate ester + NH2-----SH -----> saccharide----SH + SH-Prot (native Protein with an exposed cysteine or obtained after modification of amino groups of the protein by SPDP for instance) -----> saccharide-S-S-Prot

Saccharide-OH + CNBr or CDAP ---> cyanate ester + NH2----SH -------> saccharide----SH + maleimide-Prot (modification of amino groups) ----> conjugate

Saccharide-OH + CNBr or CDAP ---> cyanate ester + NH2-----SH ---> Saccharide-SH + haloacetylated-Prot ----> Conjugate

Saccharide-COOH + EDAC + NH2-----NH2 ---> saccharide------NH2 + EDAC + COOH-Prot ----> conjugate

Saccharide-COOH + EDAC+ NH2----SH -----> saccharide----SH + SH-Prot (native Protein with an exposed cysteine or obtained after modification of amino groups of the protein by SPDP for instance) -----> saccharide-S-S-Prot

Saccharide-COOH + EDAC+ NH2----SH -----> saccharide----SH + maleimide-Prot (modification of amino groups) ----> conjugate

Saccharide-COOH + EDAC + NH2----SH ---> Saccharide-SH + haloacetylated-Prot ----> Conjugate

Saccharide-Aldehyde + NH2-----NH2 ----> saccharide---NH2 + EDAC + COOH-Prot ----> conjugate

Note: instead of EDAC above, any suitable carbodiimide may be used.

In summary, the types of protein carrier chemical group that may be generally used for coupling with a saccharide are amino groups (for instance on lysine residues), COOH groups (for instance on aspartic and glutamic acid residues) and SH groups (if accessible) (for instance on cysteine residues.

Preferably the ratio of carrier protein to *S. pneumoniae* saccharide is between 1:5 and 5:1; e.g. between 1:0.5-4:1, 1:1-3.5:1, 1.2:1-3:1, 1.5:1-2.5:1; e.g. between 1:2 and 2.5:1; 1:1 and 2:1 (w/w). In an embodiment, the majority of the conjugates, for example 6, 7, 8, 9 or more of the conjugates have a ratio of carrier protein to saccharide that is greater than 1:1, for example 1.1:1, 1.2:1, 1.3:1, 1.4:1, 1.5:1 or 1.6:1.

In an embodiment, at least one *S. pneumoniae* saccharide is conjugated to a carrier protein via a linker (e.g. ADH) using CDAP and EDAC. For example, 18C may be conjugated to a protein via a linker (for example those with two hydrazino groups at its ends such as ADH) using CDAP and EDAC as described above. When a linker is used, CDAP may be used to conjugate the saccharide to a linker and EDAC may then be used to conjugate the linker to a protein or, alternatively EDAC may be used first to conjugate the linker to the protein, after which CDAP may be used to conjugate the linker to the saccharide.

In general, the immunogenic composition of the invention may comprise a dose of each saccharide conjugate between 0.1 and 20µg, 1 and 10µg or 1 and 3µg of saccharide.

In an embodiment, the immunogenic composition of the invention contains each *S. pneumoniae* capsular saccharide at a dose of between 0.1-20µg; 0.5-10µg; 0,5- 5µg or 1-3µg of saccharide. In an embodiment, capsular saccharides may be present at different dosages, for example some capsular saccharides may be present at a dose of exactly 1µg or some capsular saccharides may be present at a dose of exactly 3µg. In an embodiment, saccharides from serotypes 3, 18C and 19F (or 4, 18C and 19F) are present at a higher dose than other saccharides. In one aspect of this embodiment, serotypes 3, 18C and 19F (or 4, 18C and 19F) are present at a dose of around or exactly 3 µg whilst other saccharides in the immunogenic composition of the invention are present at a dose of around or exactly 1µg.

"Around" or "approximately" are defined as within 10% more or less of the given figure for the purposes of the invention.

In an embodiment, at least one of the *S. pneumoniae* capsular saccharide is directly conjugated to a carrier protein (e.g. using one of the chemistries described above); Preferably the at least one of the *S. pneumoniae* capsular saccharides is directly conjugated by CDAP. In an embodiment, the majority of the capsular saccharides for example 5, 6, 7, 8, 9 or more are directly linked to the carrier protein by CDAP (see WO 95/08348 and WO 96/29094)

The immunogenic composition may comprise *Streptococcus pneumoniae* proteins, herein termed *Streptococcus pneumoniae* proteins of the invention. Such proteins may be used as carrier proteins, or may be present as free proteins, or may be present both as carrier proteins and as free proteins. The *Streptococcus pneumoniae* proteins of the invention are either surface exposed, at least during part of the life cycle of the pneumococcus, or are proteins which are secreted or released by the pneumococcus. Preferably the proteins of the invention are selected from the following categories, such as proteins having a Type II Signal sequence motif of LXXC (where X is any amino acid, e.g., the polyhistidine triad family (PhtX)), choline binding proteins (CbpX), proteins having a Type I Signal sequence motif (e.g., Sp101), proteins having a LPXTG motif (where X is any amino acid, e.g., Sp128, Sp130), and toxins (e.g., Ply). Preferred examples within these categories (or motifs) are the following proteins, or immunologically functional equivalents thereof.

In one embodiment, the immunogenic composition of the invention comprises at least 1 protein selected from the group consisting of the Poly Histidine Triad family (PhtX), Choline Binding Protein family (CbpX), CbpX truncates, LytX family, LytX truncates, CbpX truncate-LytX truncate chimeric proteins (or fusions), pneumolysin (Ply), PspA, PsaA, Sp128, Sp101, Sp130, Sp125 and Sp133. In a further embodiment, the immunogenic composition comprises 2 or more proteins selected from the group consisting of the Poly Histidine Triad family (PhtX), Choline Binding Protein family (CbpX), CbpX truncates, LytX family, LytX truncates, CbpX truncate-LytX truncate chimeric proteins (or fusions), pneumolysin (Ply), PspA, PsaA, and Sp128. In one more embodiment, the immunogenic composition comprises 2 or more proteins selected from the group consisting of the Poly Histidine Triad family (PhtX), Choline Binding Protein family (CbpX), CbpX truncates, LytX family, LytX truncates, CbpX truncate-LytX truncate chimeric proteins (or fusions), pneumolysin (Ply), and Sp128.

The Pht (Poly Histidine Triad) family comprises proteins PhtA, PhtB, PhtD, and PhtE. The family is characterized by a lipidation sequence, two domains separated by a proline-rich region and several histidine triads, possibly involved in metal or nucleoside binding or enzymatic activity, (3-5) coiled-coil regions, a conserved N-terminus and a heterogeneous C terminus. It is present in all strains of pneumococci tested. Homologous proteins have also been found in other Streptococci and Neisseria. In one embodiment of the invention, the Pht protein of the invention is PhtD. It is understood, however, that the terms Pht A, B, D, and E refer to proteins having sequences disclosed in the citations below as well as naturally-occurring (and man-made) variants thereof that have a sequence homology that is at least 90% identical to the referenced proteins. Preferably it is at least 95% identical and most preferably it is 97% identical.

With regards to the PhtX proteins, PhtA is disclosed in WO 98/18930, and is also referred to Sp36. As noted above, it is a protein from the polyhistidine triad family and has the type II signal motif of LXXC. PhtD is disclosed in WO 00/37105, and is also referred to Sp036D. As noted above, it also is a protein from the polyhistidine triad family and has the type II LXXC signal motif. PhtB is disclosed in WO 00/37105, and is also referred to Sp036B. Another member of the PhtB family is the C3-Degrading Polypeptide, as disclosed in WO 00/17370. This protein also is from the polyhistidine triad family and has the type II LXXC signal motif. A preferred immunologically functional equivalent is the protein Sp42 disclosed in WO 98/18930. A PhtB truncate (approximately 79kD) is disclosed in WO99/1567 which is also considered a member of the PhtX family. PhtE is disclosed in WO00/30299 and is referred to as BVH-3. Where any Pht protein is referred to herein, it is meant that immunogenic fragments or fusions thereof of the Pht protein can be used. For example, a reference to PhtX includes immunogenic fragments or fusions thereof from any Pht protein. A reference to PhtD or PhtB is also a reference to PhtDE or PhtBE fusions as found, for example, in WO0198334.

Pneumolysin is a multifunctional toxin with a distinct cytolytic (hemolytic) and complement activation activities (Rubins et al., Am. Respi. Cit Care Med, 153:1339-1346 (1996)). The toxin is not secreted by pneumococci, but it is released upon lysis of pneumococci under the influence of autolysin. Its effects include e.g., the stimulation of the production of inflammatory cytokines by human monocytes, the inhibition of the beating of cilia on human respiratory epithelial, and the decrease of bactericidal activity and migration of neutrophils. The most obvious effect of pneumolysin is in the lysis of red blood cells, which involves binding to cholesterol. Because it is a toxin, it needs to be detoxified (i.e., non-toxic to a human when provided at a dosage suitable for protection) before it can be administered *in vivo*. Expression and cloning of wild-type or native pneumolysin is known in the art. See, for example, Walker et al. (Infect Immun, 55:1184-1189 (1987)), Mitchell et al. (Biochim Biophys Acta, 1007:67-72 (1989) and Mitchell et al (NAR, 18:4010 (1990)). Detoxification of ply can be conducted by chemical means, e.g., subject to formalin or glutaraldehyde treatment or a combination of both (WO 04081515, PCT/EP2005/010258). Such methods are well known in the art for various toxins. Alternatively, ply can be genetically detoxified. Thus, the invention encompasses derivatives of pneumococcal proteins which may be, for example, mutated proteins. The term "mutated" is used herein to mean a molecule which has undergone deletion, addition or substitution of one or more amino acids using well known techniques for site directed mutagenesis or any other conventional method. For example, as described above, a mutant ply protein may be altered so that it is biologically inactive whilst still maintaining its immunogenic epitopes, see, for example, WO90/06951, Berry et al. (Infect Immun, 67:981-985 (1999)) and WO99/03884.

As used herein, it is understood that the term "Ply" refers to mutated or detoxified pneumolysin suitable for medical use (i.e., non toxic).

Concerning the Choline Binding Protein family (CbpX), members of that family were originally identified as pneumococcal proteins that could be purified by choline-affininty chromatography. All of the choline-binding proteins are non-covalently bound to phosphorylcholine moieties of cell wall teichoic acid and membrane-associated lipoteichoic acid. Structurally, they have several regions in common over the entire family, although the exact nature of the proteins (amino acid sequence, length, etc.) can vary. In general, choline binding proteins comprise an N terminal region (N), conserved repeat regions (R1 and/or R2), a proline rich region (P) and a conserved choline binding region (C), made up of multiple repeats, that comprises approximately one half of the protein. As used in this application, the term "Choline Binding Protein family (CbpX)" is selected from the group consisting of Choline Binding Proteins as identified in WO97/41151, PbcA, SpsA, PspC, CbpA, CbpD, and CbpG. CbpA is disclosed in WO97/41151. CbpD and CbpG are disclosed in WO00/29434. PspC is disclosed in WO97/09994. PbcA is disclosed in WO98/21337.SpsA is a Choline binding protein disclosed in WO 98/39450. Preferably the Choline Binding Proteins are selected from the group consisting of CbpA, PbcA, SpsA and PspC.

Another preferred embodiment is CbpX truncates wherein "CbpX" is defined above and "truncates" refers to CbpX proteins lacking 50% or more of the Choline binding region (C). Preferably such proteins lack the entire choline binding region. More preferably, the such protein truncates lack (i) the choline binding region and (ii) a portion of the N-terminal half of the protein as well, yet retain at least one repeat region (R1 or R2). More preferably still, the truncate has 2 repeat regions (R1 and R2). Examples of such preferred embodiments are NR1xR2 and R1xR2 as illustrated in WO99/51266 or WO99/51188, however, other choline binding proteins lacking a similar choline binding region are also contemplated within the scope of this invention.

The LytX family is membrane associated proteins associated with cell lysis. The N-terminal domain comprises choline binding domain(s), however the LytX family does not have all the features found in the CbpA family noted above and thus for the present invention, the LytX family is considered distinct from the CbpX family. In contrast with the CbpX family, the C-terminal domain contains the catalytic domain of the LytX protein family. The family comprises LytA, B and C. With regards to the LytX family, LytA is disclosed in Ronda et al., Eur J Biochem, 164:621-624 (1987). LytB is disclosed in WO 98/18930, and is also referred to as Sp46. LytC is also disclosed in WO 98/18930, and is also referred to as Sp91. A preferred member of that family is LytC.

Another preferred embodiment are LytX truncates wherein "LytX" is defined above and "truncates" refers to LytX proteins lacking 50% or more of the Choline binding region. Preferably such proteins lack the entire choline binding region. Yet another preferred embodiment of this invention are CbpX truncate-LytX truncate chimeric proteins (or fusions). Preferably this comprises NR1xR2 (or R1xR2) of CbpX and the C-terminal portion (Cterm, i.e., lacking the choline binding domains) of LytX (e.g., LytCCterm or Sp91 Cterm). More preferably CbpX is selected from the group consisting of CbpA, PbcA, SpsA and PspC. More preferably still, it is CbpA. Preferably, LytX is LytC (also referred to as Sp91). Another embodiment of the present invention is a PspA or PsaA truncates lacking the choline binding domain (C) and expressed as a fusion protein with LytX. Preferably, LytX is LytC.

With regards to PsaA and PspA, both are know in the art. For example, PsaA and transmembrane deletion variants thereof have been described by Berry & Paton, Infect Immun 1996 Dec;64(12):5255-62. PspA and transmembrane deletion variants thereof have been disclosed in, for example, US 5804193, WO 92/14488, and WO 99/53940.

Sp128 and Sp130 are disclosed in WO00/76540. Sp125 is an example of a pneumococcal surface protein with the Cell Wall Anchored motif of LPXTG (where X is any amino acid). Any protein within this class of pneumococcal surface protein with this motif has been found to be useful within the context of this invention, and is therefore considered a further protein of the invention. Sp125 itself is disclosed in WO 98/18930, and is also known as ZmpB - a zinc metalloproteinase. Sp101 is disclosed in WO 98/06734 (where it has the reference # y85993). It is characterized by a Type I signal sequence. Sp133 is disclosed in WO 98/06734 (where it has the reference # y85992). It is also characterized by a Type I signal sequence.

Examples of preferred *Moraxella catarrhalis* protein antigens which can be included in a combination vaccine (especially for the prevention of otitis media) are: OMP106 [WO 97/41731 (Antex) & WO 96/34960 (PMC)]; OMP21 or fragments thereof (WO 0018910); LbpA &/or LbpB [WO 98/55606 (PMC)]; TbpA &/or TbpB [WO 97/13785 & WO 97/32980 (PMC)]; CopB [Helminen ME, et al. (1993) Infect. Immun. 61:2003-2010]; UspA1 and/or UspA2 [WO 93/03761 (University of Texas)]; OmpCD (EP737085); HasR (PCT/EP99/03824); PilQ (PCT/EP99/03823); OMP85 (PCT/EP00/01468); lipo06 (GB 9917977.2); iipo10 (GB 9918208.1); lipo11 (GB 9918302.2); lipo18 (GB 9918038.2); P6 (PCT/EP99/03038); D15 (PCT/EP99/03822); OmplA1 (PCT/EP99/06781); Hly3 (PCT/EP99/03257); and OmpE. Examples of non-typeable *Haemophilus influenzae* antigens or fragments thereof which can be included in a combination vaccine (especially for the prevention of otitis media) include: Fimbrin protein [(US 5766608 - Ohio State Research Foundation)] and fusions comprising peptides therefrom [eg LB1(f) peptide fusions; US 5843464 (OSU) or WO 99/64067]; OMP26 [WO 97/01638 (Cortecs)]; P6 [EP 281673 (State University of New York)]; TbpA and/or TbpB; Hia; Hsf; Hin47; Hif; Hmw1; Hmw2; Hmw3; Hmw4; Hap; D15 (WO 94/12641); P2; and P5 (WO 94/26304).

The proteins of the invention may also be beneficially combined. By combined is meant that the immunogenic composition comprises all of the proteins from within the following combinations, either as carrier proteins or as free proteins or a mixture of the two. For example, in a combination of two proteins as set out hereinafter, both proteins may be used as carrier proteins, or both proteins may be present as free proteins, or both may be present as carrier and as free protein, or one may be present as a carrier protein and a free protein whilst the other is present only as a carrier protein or only as a free protein, or one may be present as a carrier protein and the other as a free protein. Where a combination of three proteins is given, similar possibilities exist. Preferred combinations include, but are not limited to, PhtD + NR1xR2, PhtD + NR1xR2-Sp91Cterm chimeric or fusion proteins, PhtD + Ply, PhtD + Sp128, PhtD + PsaA, PhtD + PspA, PhtA + NR1xR2, PhtA + NR1xR2-Sp91 Cterm chimeric or fusion proteins, PhtA + Ply, PhtA + Sp128, PhtA + PsaA, PhtA + PspA, NR1xR2 + LytC, NR1xR2 + PspA, NR1xR2 + PsaA, NR1xR2 + Sp128, R1xR2 + LytC, R1xR2 + PspA, R1xR2 + PsaA, R1xR2 + Sp128, R1xR2 + PhtD, R1xR2 + PhtA. Preferably, NR1xR2 (or R1xR2) is from CbpA or PspC. More preferably it is from CbpA. Other combinations include 3 protein combinations such as PhtD + NR1xR2 + Ply, and PhtA + NR1xR2 + PhtD. In one embodiment, the vaccine composition comprises detoxified pneumolysin and PhtD or PhtDE as carrier proteins. In a further embodiment, the vaccine composition comprises detoxified pneumolysin and PhtD or PhtDE as free proteins.

The present invention further provides a vaccine containing the immunogenic compositions of the invention and a pharmaceutically acceptable excipient.

The vaccines of the present invention may be adjuvanted, particularly when intended for use in an elderly population. Suitable adjuvants include an aluminum salt such as aluminum hydroxide gel (alum) or aluminum phosphate, but may also be a salt of calcium, magnesium, iron or zinc, or may be an insoluble suspension of acylated tyrosine, or acylated sugars, cationically or anionically derivatized saccharides, or polyphosphazenes.

It is preferred that the adjuvant be selected to be a preferential inducer of a TH1 type of response. Such high levels of Th1-type cytokines tend to favour the induction of cell mediated immune responses to a given antigen, whilst high levels of Th2-type cytokines tend to favour the induction of humoral immune responses to the antigen.

The distinction of Th1 and Th2-type immune response is not absolute. In reality an individual will support an immune response which is described as being predominantly Th1 or predominantly Th2. However, it is often convenient to consider the families of cytokines in terms of that described in murine CD4 +ve T cell clones by Mosmann and Coffman (Mosmann, T.R. and Coffman, R.L. (1989) TH1 and TH2 cells: different patterns of lymphokine secretion lead to different functional properties. (Annual Review of Immunology, 7, p145-173). Traditionally, Th1-type responses are associated with the production of the INF-γ and IL-2 cytokines by T-lymphocytes. Other cytokines often directly associated with the induction of Th1-type immune responses are not produced by T-cells, such as IL-12. In contrast, Th2-type responses are associated with the secretion of II-4, IL-5, IL-6, IL-10. Suitable adjuvant systems which promote a predominantly Th1 response include: Monophosphoryl lipid A or a derivative thereof (or detoxified lipid A in general - see for instance WO 2005107798), particularly 3-de-O-acylated monophosphoryl lipid A (3D-MPL) (for its preparation see GB 2220211 A); and a combination of monophosphoryl lipid A, preferably 3-de-O-acylated monophosphoryl lipid A, together with either an aluminum salt (for instance aluminum phosphate or aluminum hydroxide) or an oil-in-water emulsion. In such combinations, antigen and 3D-MPL are contained in the same particulate structures, allowing for more efficient delivery of antigenic and immunostimulatory signals. Studies have shown that 3D-MPL is able to further enhance the immunogenicity of an alum-adsorbed antigen [Thoelen et al. Vaccine (1998) 16:708-14; EP689454-B1].

An enhanced system involves the combination of a monophosphoryl lipid A (or detoxified lipid A) and a saponin derivative, particularly the combination of QS21 and 3D-MPL as disclosed in WO 94/00153, or a less reactogenic composition where the QS21 is quenched with cholesterol as disclosed in WO 96/33739. A particularly potent adjuvant formulation involving QS21, 3D-MPL (or detoxified lipid A) and tocopherol in an oil in water emulsion is described in WO 95/17210. In one embodiment the immunogenic composition additionally comprises a saponin, which may be QS21. The formulation may also comprise an oil in water emulsion and tocopherol (WO 95/17210). Unmethylated CpG containing oligonucleotides (WO 96/02555) and other immunomodulatory oligonucleotides (WO0226757 and WO03507822) are also preferential inducers of a TH1 response and are suitable for use in the present invention.

Particular adjuvants are those selected from the group of metal Salts, oil in water emulsions, Toll like receptors agonist, (in particular Toll like receptor 2 agonist, Toll like receptor 3 agonist, Toll like receptor 4 agonist, Toll like receptor 7 agonist, Toll like receptor 8 agonist and Toll like receptor 9 agonist), saponins or combinations thereof.

An adjuvant that can be used with the vaccine compositions of the invention are bleb or outer membrane vesicle preparations from Gram negative bacterial strains such as those taught by WO02/09746 - particularly *N. meningitidis* blebs. Adjuvant properties of blebs can be improved by retaining LOS (lipooligosacccharide) on its surface (e.g. through extraction with low concentrations of detergent [for instanct 0-0.1% deoxycholate]). LOS can be detoxified through the msbB(-) or htrB(-) mutations discussed in WO02/09746. Adjuvant properties can also be improved by retaining PorB (and optionally removing PorA) from meningococcal blebs. Adjuvant properties can also be improved by truncating the outer core saccharide structure of LOS on meningococcal blebs - for instance via the IgtB(-) mutation discussed in WO2004/014417. Alternatively, the aforementioned LOS (e.g. isolated from a msbB(-) and/or IgtB(-) strain) can be purified and used as an adjuvant in the compositions of the invention.

A further adjuvant which may be used with the compositions of the invention may be selected from the group: a saponin, lipid A or a derivative thereof, an immunostimulatory oligonucleotide, an alkyl glucosaminide phosphate, an oil in water emulsion or combinations thereof. A further preferred adjuvant is a metal salt in combination with another adjuvant. It is preferred that the adjuvant is a Toll like receptor agonist in particular an agonist of a Toll like receptor 2, 3, 4, 7, 8 or 9, or a saponin, in particular Qs21. It is further preferred that the adjuvant system comprises two or more adjuvants from the above list. In particular the combinations preferably contain a saponin (in particular Qs21) adjuvant and/or a Toll like receptor 9 agonist such as a CpG containing immunostimulatory oligonucleotide. Other preferred combinations comprise a saponin (in particular QS21) and a Toll like receptor 4 agonist such as monophosphoryl lipid A or its 3 deacylated derivative, 3D-MPL, or a saponin (in particular QS21) and a Toll like receptor 4 ligand such as an alkyl glucosaminide phosphate.

Particularly preferred adjuvants are combinations of 3D-MPL and QS21 (EP 0 671 948 B1), oil in water emulsions comprising 3D-MPL and QS21 (WO 95/17210, WO 98/56414), or 3D-MPL formulated with other carriers (EP 0 689 454 B1). Other preferred adjuvant systems comprise a combination of 3 D MPL , QS21 and a CpG oligonucleotide as described in US6558670, US6544518.

In an embodiment the adjuvant is (or comprises) a Toll like receptor (TLR) 4 ligand, preferably an agonist such as a lipid A derivative particularly monophosphoryl lipid A or more particularly 3 Deacylated monophoshoryl lipid A (3 D-MPL).

3 D -MPL is available from GlaxoSmithKline Biologicals North America and primarily promotes CD4+ T cell responses with an IFN-g (Th1) phenotype. It can be produced according to the methods disclosed in GB 2 220 211 A. Chemically it is a mixture of 3-deacylated monophosphoryl lipid A with 3, 4, 5 or 6 acylated chains. Preferably in the compositions of the present invention small particle 3 D- MPL is used. Small particle 3 D -MPL has a particle size such that it may be sterile-filtered through a 0.22µm filter. Such preparations are described in International Patent Application No. WO 94/21292. Synthetic derivatives of lipid A are known and thought to be TLR 4 agonists including, but not limited to:
OM174 (2-deoxy-6-o-[2-deoxy-2-[(R)-3-dodecanoyloxytetra-decanoyiamino]-4-o-phosphono-β-D-glucopyranosyl]-2-[(R)-3-hydroxytetradecanoylamino]-α-D-glucopyranosyldihydrogenphosphate), (WO 95/14026)
OM 294 DP (3S, 9 R) -3--[(R)-dodecanoyloxytetradecanoylamino]-4-oxo-5-aza-9(R)-[(R)-3-hydroxytetradecanoylamino]decan-1,10-dio1,1,10-bis(dihydrogenophosphate) (WO99 /64301 and WO 00/0462)
OM 197 MP-Ac DP (3S-, 9R) -3-[(R) -dodecanoyloxytetradecanoylamino]-4-oxo-5-aza-9-[(R)-3-hydroxytetradecanoylamino]decan-1,10-diol,1-dihydrogenophosphate 10-(6-aminohexanoate) (WO 01/46127)
Other TLR4 ligands which may be used are alkyl Glucosaminide phosphates (AGPs) such as those disclosed in WO9850399 or US6303347 (processes for preparation of AGPs are also disclosed), or pharmaceutically acceptable salts of AGPs as disclosed in US6764840. Some AGPs are TLR4 agonists, and some are TLR4 antagonists. Both are thought to be useful as adjuvants.

Another prefered immunostimulant for use in the present invention is Quil A and its derivatives. Quil A is a saponin preparation isolated from the South American tree Quilaja Saponaria Molina and was first described as having adjuvant activity by Dalsgaard et al. in 1974 ("Saponin adjuvants", Archiv. für die gesamte Virusforschung, Vol. 44, Springer Verlag, Berlin, p243-254). Purified fragments of Quil A have been isolated by HPLC which retain adjuvant activity without the toxicity associated with Quil A (EP 0 362 278), for example QS7 and QS21 (also known as QA7 and QA21). QS-21 is a natural saponin derived from the bark of Quillaja saponaria Molina which induces CD8+ cytotoxic T cells (CTLs), Th1 cells and a predominant IgG2a antibody response and is a preferred saponin in the context of the present invention.

Particular formulations of QS21 have been described which are particularly preferred, these formulations further comprise a sterol (WO96/33739). The saponins forming part of the present invention may be separate in the form of micelles, mixed micelles (preferentially, but not exclusively with bile salts) or may be in the form of ISCOM matrices (EP 0 109 942 B1), liposomes or related colloidal structures such as worm-like or ring-like multimeric complexes or lipidic/layered structures and lamellae when formulated with cholesterol and lipid, or in the form of an oil in water emulsion (for example as in WO 95/17210). The saponins may preferably be associated with a metallic salt, such as aluminium hydroxide or aluminium phosphate (WO 98/15287).
Preferably, the saponin is presented in the form of a liposome, ISCOM or an oil in water emulsion.

An enhanced system involves the combination of a monophosphoryl lipid A (or detoxified lipid A) and a saponin derivative, particularly the combination of QS21 and 3D-MPL as disclosed in WO 94/00153, or a less reactogenic composition where the QS21 is quenched with cholesterol as disclosed in WO 96/33739. A particularly potent adjuvant formulation involving tocopherol with or without QS21 and/or 3D-MPL in an oil in water emulsion is described in WO 95/17210. In one embodiment the immunogenic composition additionally comprises a saponin, which may be QS21.

Immunostimulatory oligonucleotides or any other Toll-like receptor (TLR) 9 agonist may also be used.The preferred oligonucleotides for use in adjuvants or vaccines of the present invention are CpG containing oligonucleotides, preferably containing two or more dinucleotide CpG motifs separated by at least three, more preferably at least six or more nucleotides. A CpG motif is a Cytosine nucleotide followed by a Guanine nucleotide. The CpG oligonucleotides of the present invention are typically deoxynucleotides. In a preferred embodiment the internucleotide in the oligonucleotide is phosphorodithioate, or more preferably a phosphorothioate bond, although phosphodiester and other internucleotide bonds are within the scope of the invention. Also included within the scope of the invention are oligonucleotides with mixed internucleotide linkages. Methods for producing phosphorothioate oligonucleotides or phosphorodithioate are described in US5,666,153, US5,278,302 and WO95/26204.

Examples of preferred oligonucleotides have the following sequences. The sequences preferably contain phosphorothioate modified internucleotide linkages.
OLIGO 1(SEQ ID NO:1): TCC ATG ACG TTC CTG ACG TT (CpG 1826)
OLIGO 2 (SEQ ID NO:2): TCT CCC AGC GTG CGC CAT (CpG 1758)
OLIGO 3(SEQ ID NO:3): ACC GAT GAC GTC GCC GGT GAC GGC ACC ACG
OLIGO 4 (SEQ ID NO:4): TCG TCG TTT TGT CGT TTT GTC GTT (CpG 2006)
OLIGO 5 (SEQ ID NO:5): TCC ATG ACG TTC CTG ATG CT (CpG 1668)
OLIGO 6 (SEQ ID NO:6): TCG ACG TTT TCG GCG CGC GCC G (CpG 5456)

Alternative CpG oligonucleotides may comprise the preferred sequences above in that they have inconsequential deletions or additions thereto.
The CpG oligonucleotides utilised in the present invention may be synthesized by any method known in the art (for example see EP 468520). Conveniently, such oligonucleotides may be synthesized utilising an automated synthesizer.

The adjuvant may be an oil in water emulsion or may comprise an oil in water emulsion in combination with other adjuvants. The oil phase of the emulsion system preferably comprises a metabolisable oil. The meaning of the term metabolisable oil is well known in the art. Metabolisable can be defined as "being capable of being transformed by metabolism" (Dorland's Illustrated Medical Dictionary, W.B. Sanders Company, 25th edition (1974)). The oil may be any vegetable oil, fish, oil, animal or synthetic oil, which is not toxic to the recipient and is capable of being transformed by metabolism. Nuts, seeds, and grains are common sources of vegetable oils. Synthetic oils are also part of this invention and can include commercially available oils such as NEOBEE® and others. Squalene (2,6,10,15,19, 23-Hexamethyl-2,6,10,14,18,22-tetracosahexaene) is an unsaturated oil which is found in large quantities in shark-liver oil, and in lower quantities in olive oil, wheat germ oil, rice bran oil, and yeast, and is a particularly preferred oil for use in this invention. Squalene is a metabolisable oil by virtue of the fact that it is an intermediate in the biosynthesis of cholesterol (Merck index, 10^{th} Edition, entry no.8619). Tocols (e.g. vitamin E) are also often used in oil emulsions adjuvants (EP 0 382 271 B1; US5667784; WO 95/17210). Tocols used in the oil emulsions (preferably oil in water emulsions) of the invention may be formulated as described in EP 0 382 271 B1, in that the tocols may be dispersions of tocol droplets, optionally comprising an emulsifier, of preferably less than 1 micron in diameter. Alternatively, the tocols may be used in combination with another oil, to form the oil phase of an oil emulsion. Examples of oil emulsions which may be used in combination with the tocol are described herein, such as the metabolisable oils described above.

Oil in water emulsion adjuvants per se have been suggested to be useful as adjuvant compositions (EP 0 399 843B), also combinations of oil in water emulsions and other active agents have been described as adjuvants for vaccines (WO 95/17210; WO 98/56414; WO 99/12565; WO 99/11241). Other oil emulsion adjuvants have been described, such as water in oil emulsions (US 5,422,109;EP 0 480 982 B2) and water in oil in water emulsions (US 5,424,067;EP 0 480 981 B). All of which form preferred oil emulsion systems (in particular when incorporating tocols) to form adjuvants and compositions of the present invention.

Most preferably the oil emulsion (for instance oil in water emulsions) further comprises an emulsifier such as TWEEN 80 and/or a sterol such as cholesterol.
A preferred oil emulsion (preferably oil-in-water emulsion) comprises a metabolisible, non-toxic oil, such as squalane, squalene or a tocopherol such as alpha tocopherol (and preferably both squalene and alpha tocopherol) and optionally an emulsifier (or surfactant) such as Tween 80. A sterol (preferably cholesterol) may also be included.
The method of producing oil in water emulsions is well known to the man skilled in the art. Commonly, the method comprises mixing the tocol-containing oil phase with a surfactant such as a PBS/TWEEN80™ solution, followed by homogenisation using a homogenizer, it would be clear to a man skilled in the art that a method comprising passing the mixture twice through a syringe needle would be suitable for homogenising small volumes of liquid. Equally, the emulsification process in microfluidiser (M110S Microfluidics machine, maximum of 50 passes, for a period of 2 minutes at maximum pressure input of 6 bar (output pressure of about 850 bar)) could be adapted by the man skilled in the art to produce smaller or larger volumes of emulsion. The adaptation could be achieved by routine experimentation comprising the measurement of the resultant emulsion until a preparation was achieved with oil droplets of the required diameter.
In an oil in water emulsion, the oil and emulsifier should be in an aqueous carrier. The aqueous carrier may be, for example, phosphate buffered saline.

The size of the oil droplets found within the stable oil in water emulsion are preferably less than 1 micron, may be in the range of substantially 30-600nm, preferably substantially around 30-500nm in diameter, and most preferably substantially 150-500nm in diameter, and in particular about 150 nm in diameter as measured by photon correlation spectroscopy. In this regard, 80% of the oil droplets by number should be within the preferred ranges, more preferably more than 90% and most preferably more than 95% of the oil droplets by number are within the defined size ranges. The amounts of the components present in the oil emulsions of the present invention are conventionally in the range of from 0.5-20% or 2 to 10% oil (of the total dose volume), such as squalene; and when present, from 2 to 10% alpha tocopherol; and from 0.3 to 3% surfactant, such as polyoxyethylene sorbitan monooleate. Preferably the ratio of oil (preferably squalene): tocol (preferably α-tocopherol) is equal or less than 1 as this provides a more stable emulsion. An emulsifier, such as Tween80 or Span 85 may also be present at a level of about 1%. In some cases it may be advantageous that the vaccines of the present invention will further contain a stabiliser.

Examples of preferred emulsion systems are described in WO 95/17210, WO 99/11241 and WO 99/12565 which disclose emulsion adjuvants based on squalene, α-tocopherol, and TWEEN 80, optionally formulated with the immunostimulants QS21 and/or 3D-MPL. Thus in a particularly, preferred embodiment of the present invention, the adjuvant of the invention may additionally comprise further immunostimulants, such as LPS or derivatives thereof, and/or saponins. Examples of further immunostimulants are described herein and in "Vaccine Design - The Subunit and Adjuvant Approach" 1995, Pharmaceutical Biotechnology, Volume 6, Eds. Powell, M.F., and Newman, M.J., Plenum Press, New York and London, ISBN 0-306-44867-X.

In a preferred aspect the adjuvant and immunogenic compositions according to the invention comprise a saponin (preferably QS21) and/or an LPS derivative (preferably 3D-MPL) in an oil emulsion described above, optionally with a sterol (preferably cholesterol). Additionally the oil emulsion (preferably oil in water emulsion) may contain span 85 and/or lecithin and/or tricaprylin. Adjuvants comprising an oil-in-water emulsion, a sterol and a saponin are described in WO 99/12565.

Typically for human administration the saponin (preferably QS21) and/or LPS derivative (preferably 3D-MPL) will be present in a human dose of immunogenic composition in the range of 1µg - 200µg, such as 10-100µg, preferably 10µg - 50µg per dose. Typically the oil emulsion (preferably oil in water emulsion) will comprise from 2 to 10% metabolisible oil. Preferably it will comprise from 2 to 10% squalene, from 2 to 10% alpha tocopherol and from 0.3 to 3% (preferably 0.4 - 2%) emulsifier (preferably tween 80 [polyoxyethylene sorbitan monooleate]). Where both squalene and alpha tocopherol are present, preferably the ratio of squalene: alpha tocopherol is equal to or less than 1 as this provides a more stable emulsion. Span 85 (Sorbitan trioleate) may also be present at a level of 0.5 to 1% in the emulsions used in the invention. In some cases it may be advantageous that the immunogenic compositions and vaccines of the present invention will further contain a stabiliser, for example other emulsifiers/surfactants, including caprylic acid (merck index 10^{th} Edition, entry no. 1739), of which Tricaprylin is particularly preferred.

Where squalene and a saponin (preferably QS21) are included, it is of benefit to also include a sterol (preferably cholesterol) to the formulation as this allows a reduction in the total level of oil in the emulsion. This leads to a reduced cost of manufacture, improvement of the overall comfort of the vaccination, and also qualitative and quantitative improvements of the resultant immune responses, such as improved IFN-γ production. Accordingly, the adjuvant system of the present invention typically comprises a ratio of metabolisable oil:saponin (w/w) in the range of 200:1 to 300:1, also the present invention can be used in a "low oil" form the preferred range of which is 1:1 to 200:1, preferably 20:1 to 100:1, and most preferably substantially 48:1, this vaccine retains the beneficial adjuvant properties of all of the components, with a much reduced reactogenicity profile. Accordingly, the particularly preferred embodiments have a ratio of squalene:QS21 (w/w) in the range of 1:1 to 250:1, also a preferred range is 20:1 to 200:1, preferably 20:1 to 100:1, and most preferably substantially 48:1. Preferably a sterol (most preferably cholesterol) is also included present at a ratio of sapon:naterol as described herein.

The emulsion systems of the present invention preferably have a small oil droplet size in the sub-micron range. Most preferably the oil droplet sizes will be in the range 120 to 750 nm, and most preferably from 120-600nm in diameter.

A particularly potent adjuvant formulation (for ultimate combination with AIPO4 in the immunogenic compositions of the invention) involves a saponin (preferably QS21), an LPS derivative (preferably 3D-MPL) and an oil emulsion (preferably squalene and alpha tocopherol in an oil in water emulsion) as described in WO 95/17210 or in WO 99/12565 (in particular adjuvant formulation 11 in Example 2, Table 1).

Examples of a TLR 2 agonist include peptidoglycan or lipoprotein. Imidazoquinolines, such as Imiquimod and Resiquimod are known TLR7 agonists. Single stranded RNA is also a known TLR agonist (TLR8 in humans and TLR7 in mice), whereas double stranded RNA and poly IC (polyinosinic-polycytidylic acid - a commercial synthetic mimetic of viral RNA). are exemplary of TLR 3 agonists. 3D-MPL is an example of a TLR4 agonist whilst CPG is an example of a TLR9 agonist.

The immunogenic composition may comprise an antigen and an immunostimulant adsorbed onto a metal salt. Aluminium based vaccine formulations wherein the antigen and the immunostimulant 3-de-O-acylated monophosphoryl lipid A (3D-MPL), are adsorbed onto the same particle are described in EP 0 576 478 B1, EP 0 689 454 B1, and EP 0 633 784 B1. In these cases then antigen is first adsorbed onto the aluminium salt followed by the adsorption of the immunostimulant 3D-MPL onto the same aluminium salt particles. Such processes first involve the suspension of 3D-MPL by sonication in a water bath until the particles reach a size of between 80 and 500 nm. The antigen is typically adsorbed onto aluminium salt for one hour at room temperature under agitation. The 3D-MPL suspension is then added to the adsorbed antigen and the formulation is incubated at room temperature for 1 hour, and then kept at 4oC until use.

In another process, the immunostimulant and the antigen are on separate metal particles, as described in EP 1126876. The improved process comprises the adsorption of immunostimulant, onto a metallic salt particle, followed by the adsorption of the antigen onto another metallic salt particle, followed by the mixing of the discrete metallic particles to form a vaccine. The adjuvant for use in the present invention may be an adjuvant composition comprising an immunostimulant, adsorbed onto a metallic salt particle, characterised in that the metallic salt particle is substantially free of other antigen. Furthermore, vaccines are provided by the present invention and are characterised in that the immunostimulant is adsorbed onto particles of metallic salt which are substantially free from other antigen, and in that the particles of metallic salt which are adsorbed to the antigen are substantially free of other immunostimulant.

Accordingly, the present invention provides an adjuvant formulation comprising immunostimulant which has been adsorbed onto a particle of a metallic salt, characterised in the composition is substantially free of other antigen. Moreover, this adjuvant formulation can be an intermediate which, if such an adjuvant is used, is required for the manufacture of a vaccine. Accordingly there is provided a process for the manufacture of a vaccine comprising admixing an adjuvant composition which is one or more immunostimulants adsorbed onto a metal particle with an antigen. Preferably, the antigen has been pre-adsorbed onto a metallic salt. Said metallic salt may be identical or similar to the metallic salt which is adsorbed onto the immunostimulant. Preferably the metal salt is an aluminium salt, for example Aluminium phosphate or Aluminium hydroxide.

The present invention further provides for a vaccine composition comprising immunostimulant adsorbed onto a first particle of a metallic salt, and antigen adsorbed onto a metallic salt, characterised in that first and second particles of metallic salt are separate particles.

LPS or LOS derivatives or mutations or lipid A derivatives described herein are designed to be less toxic (e.g. 3D-MPL) than native lipopolysaccharides and are interchangeable equivalents with respect to any uses of these moieties described herein. They may be TLR4 ligands as described above. Other such derivatives are described in WO020786737, WO9850399, WO013461 WO0212258, WO03065806.

In one embodiment the adjuvant used for the compositions of the invention comprises a liposome carrier (made by known techniques from a phospholipids (such as dioleoyl phosphatidyl choline [DOPC]) and optionally a sterol [such as cholesterol]). Such liposome carriers may carry lipid A derivatives [such as 3D-MPL - see above] and/or saponins (such as QS21 - see above). In one embodiment the adjuvant comprises (per 0.5 mL dose) 0.1-10mg, 0.2-7, 0.3-5, 0.4-2, or 0.5-1 mg (e.g. 0.4-0.6, 0.9-1.1, 0.5 or 1 mg) phospholipid (for instance DOPC), 0.025-2.5, 0.05-1.5, 0.075-0.75, 0.1-0.3, or 0.125-0.25 mg (e.g. 0.2-0.3, 0.1-0.15, 0.25 or 0.125 mg) sterol (for instance cholesterol), 5-60, 10-50, or 20-30 µg (e.g. 5-15, 40-50, 10, 20, 30, 40 or 50 µg) lipid A derivative (for instance 3D-MPL), and 5-60, 10-50, or 20-30 µg (e.g. 5-15, 40-50, 10, 20, 30, 40 or 50 µg) saponin (for instance QS21).

This adjuvant is particularly suitable for elderly vaccine formulations. In one embodiment the vaccine composition comprising this adjuvant comprises saccharide conjugates derived from at least all the following serotypes: 4, 6B, 9V, 14, 18C, 19F, 23F, 1, 5, 7F (and may also comprise one or more from serotypes 3, 6A, 19A, and 22F), wherein the GMC antibody titre induced against one or more (or all) the vaccine components 4, 6B, 9V, 14, 18C, 19F and 23F is not significantly inferior to that induced by the Prevnar® vaccine in human vaccinees.

In one embodiment the adjuvant used for the compositions of the invention comprises an oil in water emulsion made from a metabolisable oil (such as squalene), an emulsifier (such as Tween 80) and optionally a tocol (such as alpha tocopherol). In one embodiment the adjuvant comprises (per 0.5 mL dose) 0.5-15, 1-13, 2-11, 4-8, or 5-6mg (e.g. 2-3, 5-6, or 10-11 mg) metabolisable oil (such as squalene), 0.1-10, 0.3-8, 0.6-6, 0.9-5, 1-4, or 2-3 mg (e.g. 0.9-1.1, 2-3 or 4-5 mg) emulsifier (such as Tween 80) and optionally 0.5-20, 1-15, 2-12, 4-10, 5-7 mg (e.g. 11-13, 5-6, or 2-3 mg) tocol (such as alpha tocopherol).

This adjuvant may optionally further comprise 5-60, 10-50, or 20-30 µg (e.g. 5-15, 40-50, 10, 20, 30, 40 or 50 µg) lipid A derivative (for instance 3D-MPL).

These adjuvants are particularly suitable for infant or elderly vaccine formulations. In one embodiment the vaccine composition comprising this adjuvant comprises saccharide conjugates derived from at least all the following serotypes: 4, 6B, 9V, 14, 18C, 19F, 23F, 1, 5, 7F (and may also comprise one or more from serotypes 3, 6A, 19A, and 22F), wherein the GMC antibody titre induced against one or more (or all) the vaccine components 4, 6B, 9V, 14, 18C, 19F and 23F is not significantly inferior to that induced by the Prevnar® vaccine in human vaccinees.

This adjuvant may optionally contain 0.025-2.5, 0.05-1.5, 0.075-0.75, 0.1-0.3, or 0.125-0.25 mg (e.g. 0.2-0.3, 0.1-0.15, 0.25 or 0.125 mg) sterol (for instance cholesterol), 5-60, 10-50, or 20-30 µg (e.g. 5-15, 40-50, 10, 20, 30, 40 or 50 µg) lipid A derivative (for instance 3D-MPL), and 5-60, 10-50, or 20-30 µg (e.g. 5-15, 40-50, 10, 20, 30, 40 or 50 µg) saponin (for instance QS21).

This adjuvant is particularly suitable for elderly vaccine formulations. In one embodiment the vaccine composition comprising this adjuvant comprises saccharide conjugates derived from at least all the following serotypes: 4, 6B, 9V, 14, 18C, 19F, 23F, 1, 5, 7F (and may also comprise one or more from serotypes 3, 6A, 19A, and 22F), wherein the GMC antibody titre induced against one or more (or all) the vaccine components 4, 6B, 9V, 14, 18C, 19F and 23F is not significantly inferior to that induced by the Prevnar® vaccine in human vaccinees.

In one embodiment the adjuvant used for the compositions of the invention comprises aluminium phosphate and a lipid A derivative (such as 3D-MPL). This adjuvant may comprise (per 0.5 mL dose) 100-750, 200-500, or 300-400 µg Al as aluminium phosphate, and 5-60, 10-50, or 20-30 µg (e.g. 5-15, 40-50, 10, 20, 30, 40 or 50 µg) lipid A derivative (for instance 3D-MPL).

This adjuvant is particularly suitable for elderly or infant vaccine formulations. In one embodiment the vaccine composition comprising this adjuvant comprises saccharide conjugates derived from at least all the following serotypes: 4, 6B, 9V, 14, 18C, 19F, 23F, 1, 5, 7F (and may also comprise one or more from serotypes 3, 6A, 19A, and 22F), wherein the GMC antibody titre induced against one or more (or all) the vaccine components 4, 6B, 9V, 14, 18C, 19F and 23F is not significantly inferior to that induced by the Prevnar® vaccine in human vaccinees.

The vaccine preparations containing immunogenic compositions of the present invention may be used to protect or treat a mammal susceptible to infection, by means of administering said vaccine via systemic or mucosal route. These administrations may include injection via the intramuscular, intraperitoneal, intradermal or subcutaneous routes; or via mucosal administration to the oral/alimentary, respiratory, genitourinary tracts. Intranasal administration of vaccines for the treatment of pneumonia or otitis media may be carried out (as nasopharyngeal carriage of pneumococci can be more effectively prevented, thus attenuating infection at its earliest stage). Although the vaccine of the invention may be administered as a single dose, components thereof may also be co-administered together at the same time or at different times (for instance pneumococcal saccharide conjugates could be administered separately, at the same time or 1-2 weeks after the administration of the any bacterial protein component of the vaccine for optimal coordination of the immune responses with respect to each other). For co-administration, the optional Th1 adjuvant may be present in any or all of the different administrations. In addition to a single route of administration, 2 different routes of administration may be used. For example, saccharides or saccharide conjugates may be administered IM (or ID) and bacterial proteins may be administered IN (or ID). In addition, the vaccines of the invention may be administered IM for priming doses and IN for booster doses.

The content of protein antigens in the vaccine will typically be in the range 1-100µg, preferably 5-50µg, most typically in the range 5 - 25µg. Following an initial vaccination, subjects may receive one or several booster immunizations adequately spaced.

Vaccine preparation is generally described in Vaccine Design ("The subunit and adjuvant approach" (eds Powell M.F. & Newman M.J.) (1995) Plenum Press New York). Encapsulation within liposomes is described by Fullerton, US Patent 4,235,877.

The vaccines of the present invention may be stored in solution or lyophilized. Preferably the solution is lyophilized in the presence of a sugar such as sucrose or lactose. It is still further preferable that they are lyophilized and extemporaneously reconstituted prior to use. Lyophilizing may result in a more stable composition (vaccine) and may possibly lead to higher antibody titers in the presence of 3D-MPL and in the absence of an aluminum based adjuvant.

In one aspect of the invention is provided a vaccine kit, comprising a vial containing an immunogenic composition of the invention, optionally in lyophilised form, and further comprising a vial containing an adjuvant as described herein. It is envisioned that in this aspect of the invention, the adjuvant will be used to reconstitute the lyophilised immunogenic composition.

Although the vaccines of the present invention may be administered by any route, administration of the described vaccines into the skin (ID) forms one embodiment of the present invention. Human skin comprises an outer "horny" cuticle, called the stratum corneum, which overlays the epidermis. Underneath this epidermis is a layer called the dermis, which in turn overlays the subcutaneous tissue. Researchers have shown that injection of a vaccine into the skin, and in particular the dermis, stimulates an immune response, which may also be associated with a number of additional advantages. Intradermal vaccination with the vaccines described herein forms a preferred feature of the present invention.

The conventional technique of intradermal injection, the "mantoux procedure", comprises steps of cleaning the skin, and then stretching with one hand, and with the bevel of a narrow gauge needle (26-31 gauge) facing upwards the needle is inserted at an angle of between 10-15°. Once the bevel of the needle is inserted, the barrel of the needle is lowered and further advanced whilst providing a slight pressure to elevate it under the skin. The liquid is then injected very slowly thereby forming a bleb or bump on the skin surface, followed by slow withdrawal of the needle.

More recently, devices that are specifically designed to administer liquid agents into or across the skin have been described, for example the devices described in WO 99/34850 and EP 1092444, also the jet injection devices described for example in WO 01/13977; US 5,480,381, US 5,599,302, US 5,334,144, US 5,993,412, US 5,649,912, US 5,569,189, US 5,704,911, US 5,383,851, US 5,893,397, US 5,466,220, US 5,339,163, US 5,312,335, US 5,503,627, US 5,064,413, US 5,520, 639, US 4,596,556, US 4,790,824, US 4,941,880, US 4,940,460, WO 97/37705 and WO 97/13537. Alternative methods of intradermal administration of the vaccine preparations may include conventional syringes and needles, or devices designed for ballistic delivery of solid vaccines (WO 99/27961), or transdermal patches (WO 97/48440; WO 98/28037); or applied to the surface of the skin (transdermal or transcutaneous delivery WO 98/20734 ; WO 98/28037).

When the vaccines of the present invention are to be administered to the skin, or more specifically into the dermis, the vaccine is in a low liquid volume, particularly a volume of between about 0.05 ml and 0.2 ml.

The content of antigens in the skin or intradermal vaccines of the present invention may be similar to conventional doses as found in intramuscular vaccines (see above). However, it is a feature of skin or intradermal vaccines that the formulations may be "low dose". Accordingly the protein antigens in "low dose" vaccines are preferably present in as little as 0.1 to 10µg, preferably 0.1 to 5 µg per dose; and the saccharide (preferably conjugated) antigens may be present in the range of 0.01-1µg, and preferably between 0.01 to 0.5 µg of saccharide per dose.

As used herein, the term "intradermal delivery" means delivery of the vaccine to the region of the dermis in the skin. However, the vaccine will not necessarily be located exclusively in the dermis. The dermis is the layer in the skin located between about 1.0 and about 2.0 mm from the surface in human skin, but there is a certain amount of variation between individuals and in different parts of the body. In general, it can be expected to reach the dermis by going 1.5 mm below the surface of the skin. The dermis is located between the stratum corneum and the epidermis at the surface and the subcutaneous layer below. Depending on the mode of delivery, the vaccine may ultimately be located solely or primarily within the dermis, or it may ultimately be distributed within the epidermis and the dermis.

The present invention further provides an improved vaccine for the prevention or amelioration of Otitis media caused by *Haemophilus influenzae* by the addition of *Haemophilus influenzae* proteins (see above), for example protein D in free or conjugated form. In addition, the present invention further provides an improved vaccine for the prevention or amelioration of pneumococcal infection in infants (e.g., Otitis media), by relying on the addition of one or two pneumococcal proteins (see above) as free or conjugated protein to the *S. pneumoniae* conjugate compositions of the invention. Said pneumococcal free proteins may be the same or different to any *S. pneumoniae* proteins used as carrier proteins. One or more *Moraxella catarrhalis* protein antigens (see above) can also be included in the combination vaccine in a free or conjugated form. Thus, the present invention is an improved method to elicit a (protective) immune response against Otitis media in infants.

In another embodiment, the present invention is an improved method to elicit a (protective) immune response in infants (0-2 years old) by administering a safe and effective amount of the vaccine of the invention. Further embodiments of the present invention include the provision of the antigenic *S. pneumoniae* conjugate compositions of the invention for use in medicine and the use of the *S. pneumoniae* conjugates of the invention in the manufacture of a medicament for the prevention (or treatment) of pneumococcal disease.

In yet another embodiment, the present invention is an improved method to elicit a (protective) immune response in the elderly population (in the context of the present invention a patient is considered elderly if they are 50 years or over in age, typically over 55 years and more generally over 60 years) by administering a safe and effective amount of the vaccine of the invention, preferably in conjunction with one or two *S. pneumoniae* proteins present as free or conjugated protein, which free *S. pneumoniae* proteins may be the same or different as any *S. pneumoniae* proteins used as carrier proteins.

A further aspect of the invention is a method of immunising a human host against disease caused by *S. pneumoniae* and optionally *Haemophilus influenzae* infection comprising administering to the host an immunoprotective dose of the immunogenic composition or vaccine or kit of the invention.

A further aspect of the invention is an immunogenic composition of the invention for use in the treatment or prevention of disease caused by *S.pneumoniae* and optionally *Haemophilus influenzae* infection.

A further aspect of the invention is use of the immunogenic composition or vaccine or kit of the invention in the manufacture of a medicament for the treatment or prevention of diseases caused by *S. pneumoniae* and optionally *Haemophilus influenzae* infection.

In a further aspect of the invention there is provided a use of an immunogenic composition or vaccine of the invention in the manufacture of a medicament for the treatment or prevention of diseases caused by *Streptococcus pneumoniae* infection by O-acetylated serotype 18C strains and de-O-Acetylated serotype 18C strains.

In a further aspect of the invention there is provided a use of an immunogenic composition or vaccine of the invention comprising a capsular saccharide conjugate of serotype 19F but not comprising capsular saccharide from serotype 19A in the manufacture of a medicament for the treatment or prevention of diseases caused by *Streptococcus pneumoniae* infection by serotype 19A strains.

The terms "comprising", "comprise" and "comprises" herein are intended by the inventors to be optionally substitutable with the terms "consisting of", "consist of" and "consists of", respectively, in every instance.

Embodiments herein relating to "vaccine compositions" of the invention are also applicable to embodiments relating to "immunogenic compositions" of the invention, and vice versa.

Further embodiments of the invention will now be described within the following numbered paragraphs:
**Paragraph 1.** A *Streptococcus pneumoniae* immunogenic composition comprising 9 or more, 10 or more, 11 or more, or 13 or more capsular saccharides from different S. *pneumoniae* serotypes which are conjugated to a carrier protein, wherein the composition comprises conjugated capsular saccharide 18C which is less than 80, 70, 60, 50, 40, 30, 20, 15 or 10 % O-Acetylated.
**Paragraph 2.** The immunogenic composition of Paragraph 1, wherein the conjugated capsular saccharide 18C is not conjugated to pneumolysin.
**Paragraph 3.** The immunogenic composition of Paragraph 1 or 2, wherein the conjugated capsular saccharide 18C is not conjugated to CRM197.
**Paragraph 4.** The immunogenic composition of Paragraphs 1-3, wherein the conjugated capsular saccharide 18C is not conjugated with reductive amination chemistry.
**Paragraph 5.** The immunogenic composition of Paragraphs 1-3, wherein the conjugated capsular saccharide 18C is conjugated with reductive amination chemistry.
**Paragraph 6.** The immunogenic composition of Paragraphs 1-5, wherein the capsular saccharide 18C is de-O-Acetylated through treatment with acid before its conjugation.
**Paragraph 7.** An immunogenic composition according to any preceding Paragraph wherein the capsular saccharide 18C is conjugated to TT, optionally wherein 18C is the only *S. pneumoniae* capsular saccharide conjugated to TT.
**Paragraph 8.** The immunogenic composition of any preceding Paragraph, wherein the capsular saccharides from different *S. pneumoniae* serotypes are conjugated to 2 or more different carrier proteins.
**Paragraph 9.** An immunogenic composition according to any preceding Paragraph wherein 18C capsular saccharide is directly conjugated to the carrier protein.
**Paragraph 10.** An immunogenic composition of Paragraph 1 to 8 wherein 18C capsular saccharide is conjugated to the carrier protein via a linker.
**Paragraph 11.** An immunogenic composition according to Paragraph 10 wherein the linker is bifunctional.
**Paragraph 12.** The immunogenic composition of Paragraph 10 or 11 wherein the linker is ADH.
**Paragraph 13.** The immunogenic composition of Paragraphs 10-12 wherein the linker is attached to the carrier protein by carbodiimide chemistry, preferably using EDAC.
**Paragraph 14.** The immunogenic composition of Paragraphs 10 to 13 wherein the saccharide is conjugated to the linker before the carrier protein is conjugated to the linker.
**Paragraph 15.** The immunogenic composition of any of Paragraphs 10 to 13 wherein the carrier protein is conjugated to the linker before the saccharide is conjugated to the linker.
**Paragraph 16.** The immunogenic composition of any preceding Paragraph wherein the 18C saccharide is conjugated to the carrier protein or linker using CDAP chemistry.
**Paragraph 17.** The immunogenic composition of any preceding Paragraph wherein the 18C saccharide is conjugated to the carrier protein or linker using reductive amination.
**Paragraph 18.** The immunogenic composition of any preceding Paragraph wherein the ratio of carrier protein to 18C saccharide is between 1:5 and 5:1, 1:1 and 4:1, 1.5:1 and 3:1, or 2:1 and 2.5:1 (w/w).
**Paragraph 19.** The immunogenic composition of any preceding Paragraph wherein the M_{w} of the 18C saccharide is above 20 x10³, optionally below 5x10⁵.
**Paragraph 20.** The immunogenic composition of Paragraph 19 wherein the M_{w} of the 18C saccharide is between 20 x10³ and 100 x10³.
**Paragraph 21.** The immunogenic composition of any preceding Paragraph wherein the 18C saccharide is either a native polysaccharide or is sized.
**Paragraph 22.** The immunogenic composition of Paragraph 21 wherein the 18C saccharide has been sized by microfluidization.
**Paragraph 23.** The immunogenic composition of Paragraph 21 wherein the 18C saccharide has been sized by acid hydrolysis.
**Paragraph 24.** The immunogenic composition of any preceding Paragraph wherein the dose of the 18C saccharide conjugate is between 1 and 10 µg, 1 and 5 µg, or 1 and 3 µg of saccharide.
**Paragraph 25.** The immunogenic composition of Paragraph 24 wherein the dose of the 18C saccharide conjugate is 3 µg of saccharide.
**Paragraph 26.** An immunogenic composition according to any preceding Paragraph, wherein said composition further comprises protein D from *Haemophilus influenzae,* optionally as a carrier protein for a S. *pneumoniae* capsular saccharide.
**Paragraph 27.** The *Streptococcus pneumoniae* immunogenic composition of any preceding Paragraph, wherein the vaccine comprises serotype 19F capsular saccharide conjugated to diphtheria toxoid (DT) or CRM197, optionally wherein 19F is the only saccharide in the composition conjugated to diphtheria toxoid (DT) or CRM197.
**Paragraph 28.** An immunogenic composition according to Paragraph 27 wherein serotype 19F capsular saccharide is conjugated to Diphtheria toxoid.
**Paragraph 29.** An immunogenic composition according to Paragraph 27 or 28 wherein 19F capsular saccharide is directly conjugated to the carrier protein.
**Paragraph 30.** An immunogenic composition according to Paragraph 27 or 28 wherein 19F capsular saccharide is conjugated to the carrier protein via a linker.
**Paragraph 31.** An immunogenic composition according to Paragraph 30 wherein the linker is bifunctional.
**Paragraph 32.** The immunogenic composition of Paragraph 30 or 31 wherein the linker is ADH.
**Paragraph 33.** The immunogenic composition of Paragraphs 30-32 wherein the linker is attached to the carrier protein by carbodiimide chemistry, preferably using EDAC.
**Paragraph 34.** The immunogenic composition of any of Paragraphs 30-33 wherein the saccharide is conjugated to the linker before the carrier protein is conjugated to the linker.
**Paragraph 35.** The immunogenic composition of any of Paragraphs 30-33 wherein the carrier protein is conjugated to the linker before the saccharide is conjugated to the linker.
**Paragraph 36.** The immunogenic composition of Paragraphs 27-35 wherein the 19F saccharide is conjugated to the carrier protein or to the linker using CDAP chemistry.
**Paragraph 37.** The immunogenic composition of Paragraphs 27-36 wherein the ratio of carrier protein to 19F saccharide is between 1.4 and 1.5:1 (w/w).
**Paragraph 38.** The immunogenic composition of Paragraphs 27-37 wherein the M_{w} of the 19F saccharide is above 100 x10³, and optionally below 5 x10⁵.
**Paragraph 39.** The immunogenic composition of Paragraph 38 wherein the M_{w} of the 19F saccharide is between 125 x10³ and 150 x10³.
**Paragraph 40.** The immunogenic composition of Paragraphs 27-39 wherein the 19F saccharide is either a native polysaccharide or is sized by a factor of no more than x5.
**Paragraph 41.** The immunogenic composition of Paragraphs 27-40 wherein the 19F saccharide has been sized by microfluidization.
**Paragraph 42.** The immunogenic composition of Paragraphs 27-41 wherein the dose of the 19F saccharide conjugate is between 1 and 10 µg, 1 and 5 µg, or 1 and 3 µg of saccharide.
**Paragraph 43.** The immunogenic composition of Paragraphs 27-42 wherein the 19F capsular saccharide is more than 50, 60, 70, 80, or 90% N-Acetylated.
**Paragraph 44.** An immunogenic composition according to any preceding Paragraph wherein at least 8 of the capsular saccharides are conjugated to the same carrier protein.
**Paragraph 45.** An immunogenic composition according to Paragraph 44 wherein said same carrier protein is not diphtheria toxoid and/or is not tetanus toxoid.
**Paragraph 46.** An immunogenic composition according to Paragraph 44 or 45, wherein said same carrier protein is *H. influenzae* protein D.
**Paragraph 47.** An immunogenic composition according to any preceding Paragraph comprising 2 different carrier proteins.
**Paragraph 48.** An immunogenic composition according to any preceding Paragraph comprising 3, 4 or 5 different carrier proteins.
**Paragraph 49.** An immunogenic composition according to any preceding Paragraph, wherein the composition comprises one or more or all carrier proteins is selected from the group consisting of DT, CRM197, TT, Fragment C, dPly, PhtA, PhyB, PhtD, PhtE, PhtDE, OmpC, PsaA, PspC, CbpA, PorB and *Haemophilus influenzae* Protein D.
**Paragraph 50.** An immunogenic composition according to any preceding Paragraph, wherein the composition comprises DT or CRM197 as a carrier protein.
**Paragraph 51.** An immunogenic composition according to any preceding Paragraph, wherein the composition comprises TT as a carrier protein.
**Paragraph 52.** An immunogenic composition according to any preceding Paragraph, wherein the composition comprises PD as a carrier protein.
**Paragraph 53.** An immunogenic composition according to any preceding Paragraph, wherein the composition comprises pneumolysin as a carrier protein, or alternatively as free protein.
**Paragraph 54.** An immunogenic composition according to any preceding Paragraph, wherein the composition comprises PhtA, PhtB, PhtD, PhtE or PhtDE as a carrier protein, or alternatively as free protein.
**Paragraph 55.** An immunogenic composition according to any preceding Paragraph wherein at least capsular saccharides from serotypes 4, 6B, 9V, 14, 18C, 19F and 23F are present as conjugated capsular saccharides.
**Paragraph 56.** The immunogenic composition of Paragraph 55, wherein capsular saccharide from serotype 4 is more than 50, 60, 70, 80, 90 % N-Acetylated.
**Paragraph 57.** The immunogenic composition of Paragraph 55 or 56, wherein capsular saccharide from serotype 9V is more than 50, 60, 70, 80, 90 % N-Acetylated and/or more than 50, 60, 70, 80, 90 % O-Acetylated.
**Paragraph 58.** The immunogenic composition of Paragraphs 55-57, wherein capsular saccharide from serotype 14 is more than 50, 60, 70, 80, 90 % N-Acetylated.
**Paragraph 59.** An immunogenic composition of Paragraphs 55-58 further comprising capsular saccharide from serotype 1 as conjugated capsular saccharide.
**Paragraph 60.** The immunogenic composition of Paragraph 59, wherein capsular saccharide from serotype 1 is more than 50, 60, 70, 80, 90 % N-Acetylated.
**Paragraph 61.** The immunogenic composition of Paragraph 59 or 60, wherein capsular saccharide from serotype 1 is more than 50, 60, 70, 80, 90 % O-Acetylated.
**Paragraph 62.** An immunogenic composition of Paragraphs 55-61 further comprising capsular saccharide from serotype 5 as conjugated capsular saccharide.
**Paragraph 63.** The immunogenic composition of Paragraph 62, wherein capsular saccharide from serotype 5 is more than 50, 60, 70, 80, 90 % N-Acetylated.
**Paragraph 64.** An immunogenic composition of Paragraphs 55-63 further comprising capsular saccharide from serotype 7F as conjugated capsular saccharide.
**Paragraph 65.** The immunogenic composition of Paragraph 64, wherein capsular saccharide from serotype 7F is more than 50, 60, 70, 80, 90 % N-Acetylated and/or more than 50, 60, 70, 80, 90 % O-Acetylated.
**Paragraph 66.** An immunogenic composition of Paragraphs 55-65 wherein capsular saccharides from serotypes 1, 4, 5, 6B, 7F, 9V, 14 and 23F are all conjugated to protein D.
**Paragraph 67.** An immunogenic composition according to Paragraph 55 or 66 further comprising capsular saccharide from serotype 3 present as a conjugated saccharide.
**Paragraph 68.** An immunogenic composition according to Paragraph 67 wherein capsular saccharide from serotype 3 is conjugated to protein D.
**Paragraph 69.** An immunogenic composition according to any of Paragraphs 55 to 68 further comprising conjugated capsular saccharide from serotype 6A and/or 15B.
**Paragraph 70.** An immunogenic composition according to any of Paragraphs 55 to 69 further comprising conjugated capsular saccharide from serotype 19A.
**Paragraph 71.** An immunogenic composition according to any of Paragraphs 55 to 70 further comprising conjugated capsular saccharide from serotype 22F.
**Paragraph 72.** An immunogenic composition according to any of Paragraphs 55 to 71 further comprising conjugated capsular saccharide from serotype 8.
**Paragraph 73.** An immunogenic composition according to any of Paragraphs 55 to 72 further comprising conjugated capsular saccharide from serotype 12F.
**Paragraph 74.** An immunogenic composition according to Paragraphs 55-73, wherein at least one of the capsular saccharides is directly conjugated to the carrier protein.
**Paragraph 75.** An immunogenic composition of Paragraphs 55-74, wherein at least one of the capsular saccharides is conjugated to the carrier protein via a linker.
**Paragraph 76.** An immunogenic composition according to Paragraph 75, wherein the linker is bi-functional.
**Paragraph 77.** The immunogenic composition of Paragraph 75 or 76 wherein the linker is ADH.
**Paragraph 78.** The immunogenic composition of Paragraphs 75-77 wherein the linker is attached to the carrier protein by carbodiimide chemistry, preferably using EDAC.
**Paragraph 79.** The immunogenic composition of any of Paragraphs 75 to 78 wherein the saccharide is conjugated to the linker before the carrier protein is conjugated to the linker.
**Paragraph 80.** The immunogenic composition of any of Paragraphs 75 to 78 wherein the carrier protein is conjugated to the linker before the saccharide is conjugated to the linker.
**Paragraph 81.** The immunogenic composition of any preceding Paragraph wherein at least one of the capsular saccharides is conjugated to the carrier protein and/or linker using CDAP chemistry.
**Paragraph 82.** The immunogenic composition of any preceding Paragraph wherein at least one of the capsular saccharides is conjugated to the carrier protein and/or linker using reductive amination.
**Paragraph 83.** The immunogenic composition of Paragraph 82, wherein capsular saccharide 3 is conjugated to the carrier protein and/or linker using reductive amination.
**Paragraph 84.** The immunogenic composition of Paragraph 82 or 83, wherein capsular saccharide 1 is conjugated to the carrier protein and/or linker using reductive amination.
**Paragraph 85.** The immunogenic composition of any preceding Paragraph wherein the ratio of carrier protein to saccharides is between 1:2 and 2.5:1 (w/w).
**Paragraph 86.** The immunogenic composition of any preceding Paragraph wherein the M_{w} of the saccharides is above 50 x10³, optionally less than 1 x10⁶.
**Paragraph 87.** The immunogenic composition according to Paragraph 86 which comprises serotype 1 capsular saccharide having an M_{w} of between 300 x10³ and 400 x10³.
**Paragraph 88.** The immunogenic composition according to paragraph 86 or 87 which comprises serotype 4 capsular saccharide having an M_{w} of between 75 x10³ and 125 x10³.
**Paragraph 89.** The immunogenic composition of Paragraph 86-88 which comprises serotype 5 capsular saccharide having an M_{w} of between 350 x10³ and 450 x10³.
**Paragraph 90.** The immunogenic composition of Paragraphs 86 to 89 which comprises serotype 6B capsular saccharide having an M_{w} of between 1000 x10³ and 1400 x10³.
**Paragraph 91.** The immunogenic composition according to any of Paragraphs 86 to 90 which comprises serotype 7F capsular saccharide having an M_{w} of between 200 x10³ and 300x10³.
**Paragraph 92.** The immunogenic composition according to any of Paragraphs 86 to 91 which comprises serotype 9V capsular saccharide having an M_{w} of between 250 x10³ and 300x10³
**Paragraph 93.** The immunogenic composition according to any of Paragraphs 86 to 92 which comprises serotype 14 capsular saccharide having an M_{w} of between 200 x10³ and 250x10³.
**Paragraph 94.** The immunogenic composition according to any of Paragraphs 86 to 93 which comprises serotype 23F capsular saccharide having an M_{w} of between 900 x10³ and 1000 x10³.
**Paragraph 95.** The immunogenic composition of any preceding Paragraph which comprises serotypes 5, 6B and 23F as native capsular saccharide conjugates.
**Paragraph 96.** The immunogenic composition of any preceding Paragraph wherein the dose of the capsular saccharide conjugates is between 1 and 10 µg, 1 and 5 µg, or 1 and 3 µg of saccharide per conjugate.
**Paragraph 97.** The immunogenic composition of any preceding Paragraph which comprises conjugates of capsular saccharides from serotypes 4, 18C and 19F at dosages of 3 µg of saccharide per conjugate.
**Paragraph 98.** The immunogenic composition of any preceding Paragraph which comprises conjugates of serotypes 1, 5, 6B, 7F, 9V, 14 and 23F at dosages of 1 µg of saccharide per conjugate.
**Paragraph 99.** The immunogenic composition of any preceding Paragraph which further comprises unconjugated *S. pneumoniae* saccharides of serotypes different from those conjugated, such that the number of conjugated and unconjugated saccharide serotypes is less than or equal to 23.
**Paragraph 100.** The immunogenic composition of any preceding Paragraph which further comprises one or more unconjugated or conjugated *S pneumoniae* proteins.
**Paragraph 101.** The immunogenic composition of Paragraph 100 which comprises one or more unconjugated *S. pneumoniae* proteins.
**Paragraph 102.** The immunogenic composition of Paragraph 100 or 101 wherein said one or more *S. pneumoniae* proteins are selected from Poly Histidine Triad family (PhtX), Choline Binding Protein family (CbpX), CbpX truncates, LytX family, LytX truncates, CbpX truncate-LytX truncate chimeric proteins, detoxified pneumolysin (Ply), PspA, PsaA, Sp128, Sp101, Sp130, Sp125 and Sp133.
**Paragraph 103.** The immunogenic composition of Paragraphs 100-102 which comprises pneumolysin.
**Paragraph 104.** The immunogenic composition of any of Paragraphs 101-103 which comprises a PhtX protein.
**Paragraph 105.** An immunogenic composition according to any preceding Paragraph which comprises pneumolysin as free or carrier protein (for instance for a S. pneumoniae capsular saccharide).
**Paragraph 106.** An immunogenic composition according to any preceding Paragraph which comprises a PhtX protein as free or carrier protein (for instance for a S. pneumoniae capsular saccharide).
**Paragraph 107.** The immunogenic composition of Paragraph 106 wherein said PhtX protein is PhtD or a PhtBD or PhtDE fusion protein.
**Paragraph 108.** A immunogenic composition according to any preceding Paragraph which further comprises an adjuvant.
**Paragraph 109.** An immunogenic composition according to Paragraph 108 wherein the adjuvant is a preferential inducer of a Th1 response.
**Paragraph 110.** An immunogenic composition according to Paragraph 108 or 109 wherein the adjuvant comprises one or more components selected from QS21, MPL® or an immunostimulatory oligonucleotide.
**Paragraph 111.** An immunogenic composition of Paragraphs 108 to 110 wherein the adjuvant comprises QS21 and MPL.
**Paragraph 112.** An immunogenic composition according to Paragraph 111 wherein the adjuvant is a liposomal formulation.
**Paragraph 113.** An immunogenic composition according to Paragraph 111 wherein the adjuvant is an oil in water formulation.
**Paragraph 114.** A vaccine kit comprising an immunogenic composition according to any of Paragraphs 1 to 107 and further comprising for concomitant or sequential administration an adjuvant as defined in any of Paragraphs 109 to 113.
**Paragraph 115.** A vaccine comprising the immunogenic composition of any one of Paragraphs 1 to 113 and a pharmaceutically acceptable excipient.
**Paragraph 116.** A process for making the vaccine according to Paragraph 115 which comprises the step of mixing the immunogenic composition of any of Paragraphs 1 to 113 with a pharmaceutically acceptable excipient.
**Paragraph 117.** A method of immunising a human host against disease caused by *Streptococcus pneumoniae* infection comprising administering to the host an immunoprotective dose of the immunogenic composition of any of Paragraphs 1 to 113 or a vaccine of Paragraph 115.
**Paragraph 118.** The immunogenic composition of Paragraphs 1-113 or vaccine of Paragraph 115 for use in the treatment or prevention of disease caused by *Streptococcus pneumoniae* infection.
**Paragraph 119.** The use of the immunogenic composition of Paragraphs 1-113 or vaccine of Paragraph 115 in the manufacture of a medicament for the treatment or prevention of diseases caused by *Streptococcus pneumoniae* infection.
**Paragraph 120.** The use of the immunogenic composition of Paragraphs 1-113 or vaccine of Paragraph 115 in the manufacture of a medicament for the treatment or prevention of diseases caused by *Streptococcus pneumoniae* infection by O-acetylated serotype 18C strains and de-O-Acetylated serotype 18C strains.
**Paragraph 121.** The use of the immunogenic composition of Paragraphs 1-113 or vaccine of Paragraph 115 comprising a capsular saccharide conjugate of serotype 19F but not comprising capsular saccharide from serotype 19A in the manufacture of a medicament for the treatment or prevention of diseases caused by *Streptococcus pneumoniae* infection by serotype 19A strains.
**Paragraph 122.** A method of eliciting a protective immune response in infants against Otitis media comprising the administration as separate or combined components, sequentially or concomitantly (i) an immunogenic composition or vaccine according to any of Paragraphs 1 to 115 and (ii) Protein D from *Haemophilus influenzae* which protein D may be free or conjugated.
**Paragraph 123.** A method of eliciting a protective immune response to infants against *S. pneumonia* by administering the immunogenic composition or vaccine of any preceding Paragraph.
**Paragraph 124.** A method of eliciting a protective immune response to the elderly against *S. pneumonia* by administering in combination, sequentially or concomitantly (i) the immunogenic composition or vaccine of any preceding Paragraph (ii) one or more *S. pneumoniae* surface proteins selected from the group consisting of the PhtX family and pneumolysin.
**Paragraph 125.** A method of eliciting a protective immune response to infants against Otitis media by administering the immunogenic composition or vaccine of any preceding Paragraph.
**Paragraph 126.** A method of eliciting a protective immune response to infants against Otitis media by administering as separate or combined components, sequentially or concomitantly (i) the vaccine of any preceding Paragraph (ii) one or more *S. pneumoniae* surface proteins selected from the group consisting of the PhtX family and pneumolysin.
**Paragraph 127.** A vaccine kit comprising a first container comprising a *Streptococcus pneumoniae* immunogenic composition comprising 9 or more, 10 or more, 11 or more, or 13 or more capsular saccharides from different *S. pneumoniae* serotypes which are conjugated to a carrier protein, wherein no less and no more than 1 (or 2 or 3) capsular saccharide is conjugated to tetanus toxoid, and a second container comprising a DTPa or DTPw vaccine.
**Paragraph 128.** A vaccine kit comprising a first container comprising a *Streptococcus pneumoniae* immunogenic composition comprising 9 or more, 10 or more, 11 or more, or 13 or more capsular saccharides from different *S*. *pneumoniae* serotypes which are conjugated to a carrier protein, wherein no less and no more than 1 (or 2 or 3) capsular saccharide is conjugated to tetanus toxoid, and a second container comprising MenACWY (conjugated capsular saccharides of *N. meningitidis* A, C, W135 and Y), wherein one or more or all conjugated capsular saccharides in the second container are conjugated to tetanus toxoid.
**Paragraph 129.** The vaccine kit of Paragraphs 127 or 128, wherein in the first container the capsular saccharide of serotype 18C is conjugated to tetanus toxoid.
**Paragraph 130.** The vaccine kit of Paragraphs 127-129 wherein the immunogenic composition in the first container is that of Paragraphs 1-113.
**Paragraph 131.** A method of immunising a patient with the vaccine kits of Paragraphs 127-130 comprising the step of administering concomitantly an immunoprotective amount of the vaccines of the first and second containers.

All references or patent applications cited within this patent specification are incorporated by reference herein.

In order that this invention may be better understood, the following examples are set forth. These examples are for purposes of illustration only, and are not to be construed as limiting the scope of the invention in any manner.

### Examples

### Example 1: EXPRESSION OF PROTEIN D

### Haemophilus influenzae protein D

### Genetic construction for protein D expression Starting materials

### The Protein D encoding DNA

Protein D is highly conserved among *H. influenzae* of all serotypes and non-typeable strains. The vector pHIC348 containing the DNA sequence encoding the entire protein D gene has been obtained from Dr. A. Forsgren, Department of Medical Microbiology, University of Lund, Malmö General Hospital, Malmö, Sweden. The DNA sequence of protein D has been published by Janson et al. (1991) Infect. Immun. 59: 119-125.

### The expression vector pMG1

The expression vector pMG1 is a derivative of pBR322 (Gross *et al.,* 1985) in which bacteriophage λ derived control elements for transcription and translation of foreign inserted genes were introduced (Shatzman *et al.,* 1983). In addition, the Ampicillin resistance gene was exchanged with the Kanamycin resistance gene.

### The E. coli strain AR58

The *E. coli* strain AR58 was generated by transduction of N99 with a P1 phage stock previously grown on an SA500 derivative (galE::TN10, lambdakil⁻ cl857 ΔH1). N99 and SA500 are *E*. *coli* K12 strains derived from Dr. Martin Rosenberg's laboratory at the National Institute of Health.

### The expression vector pMG 1

For the production of protein D, the DNA encoding the protein has been cloned into the expression vector pMG 1. This plasmid utilises signals from lambdaphage DNA to drive the transcription and translation of inserted foreign genes. The vector contains the promoter PL, operator OL and two utilisation sites (NutL and NutR) to relieve transcriptional polarity effects when N protein is provided (Gross *et al.,* 1985). Vectors containing the PL promoter, are introduced into an *E*. *coli* lysogenic host to stabilise the plasmid DNA. Lysogenic host strains contain replication-defective lambdaphage DNA integrated into the genome (Shatzman *et al.,* 1983). The chromosomal lambdaphage DNA directs the synthesis of the cl repressor protein which binds to the OL repressor of the vector and prevents binding of RNA polymerase to the PL promoter and thereby transcription of the inserted gene. The cl gene of the expression strain AR58 contains a temperature sensitive mutant so that PL directed transcription can be regulated by temperature shift, i.e. an increase in culture temperature inactivates the repressor and synthesis of the foreign protein is initiated. This expression system allows controlled synthesis of foreign proteins especially of those that may be toxic to the cell (Shimataka & Rosenberg, 1981).

### The E. coli strain AR58

The AR58 lysogenic *E. coli* strain used for the production of the protein D carrier is a derivative of the standard NIH *E*. *coli* K12 strain N99 (F⁻ su⁻ gaIK2, lacZ⁻ thr⁻). It contains a defective lysogenic lambdaphage (galE::TN10, lambdakil- cl857 ΔH1). The Kil⁻phenotype prevents the shut off of host macromolecular synthesis. The cl857 mutation confers a temperature sensitive lesion to the cl repressor. The ΔH1 deletion removes the lambdaphage right operon and the hosts bio, uvr3, and chlA loci. The AR58 strain was generated by transduction of N99 with a P1 phage stock previously grown on an SA500 derivative (galE::TN10, lambdakil⁻ cl857 ΔH1). The introduction of the defective lysogen into N99 was selected with tetracycline by virtue of the presence of a TN10 transposon coding for tetracyclin resistance in the adjacent galE gene.

### Construction of vector pMGMDPPrD

The pMG 1 vector which contains the gene encoding the non-structural S1 protein of Influenzae virus (pMGNSI) was used to construct pMGMDPPrD. The protein D gene was amplified by PCR from the pHIC348 vector (Janson et al. 1991 Infect. Immun. 59:119-125) with PCR primers containing Ncol and Xbal restriction sites at the 5' and 3' ends, respectively. The Ncol/Xbal fragment was then introduced into pMGNS1 between Ncol and Xbal thus creating a fusion protein containing the N-terminal 81 amino acids of the NS1 protein followed by the PD protein. This vector was labelled pMGNS1PrD.

Based on the construct described above the final construct for protein D expression was generated. A BamHI/BamHI fragment was removed from pMGNS1PrD. This DNA hydrolysis removes the NS1 coding region, except for the first three N-terminal residues. Upon religation of the vector a gene encoding a fusion protein with the following N-terminal amino acid sequence has been generated:

| -----MDP SSHSSNMANT----- | |
|---|---|
| NS1 | Protein D |

The protein D does not contain a leader peptide or the N-terminal cysteine to which lipid chains are normally attached. The protein is therefore neither excreted into the periplasm nor lipidated and remains in the cytoplasm in a soluble form.

The final construct pMG-MDPPrD was introduced into the AR58 host strain by heat shock at 37°C. Plasmid containing bacteria were selected in the presence of Kanamycin. Presence of the protein D encoding DNA insert was demonstrated by digestion of isolated plasmid DNA with selected endonucleases. The recombinant *E*. *coli* strain is referred to as ECD4.

Expression of protein D is under the control of the lambda P_{L} promoter/ O_{L} Operator. The host strain AR58 contains a temperature-sensitive cl gene in the genome which blocks expression from lambda P_{L} at low temperature by binding to O_{L}. Once the temperature is elevated cl is released from O_{L} and protein D is expressed.

### Small-scale preparation

At the end of the fermentation the cells are concentrated and frozen.

The extraction from harvested cells and the purification of protein D was performed as follows. The frozen cell culture pellet is thawed and resuspended in a cell disruption solution (Citrate buffer pH 6.0) to a final OD₆₅₀ = 60. The suspension is passed twice through a high pressure homogenizer at P = 1000 bar. The cell culture homogenate is clarified by centrifugation and cell debris is removed by filtration. In the first purification step the filtered lysate is applied to a cation exchange chromatography column (SP Sepharose Fast Flow). PD binds to the gel matrix by ionic interaction and is eluted by a step increase of the ionic strength of the elution buffer.

In a second purification step impurities are retained on an anionic exchange matrix (Q Sepharose Fast Flow). PD does not bind onto the gel and can be collected in the flow through.

In both column chromatography steps fraction collection is monitored by OD. The flow through of the anionic exchange column chromatography containing the purified protein D is concentrated by ultrafiltration.

The protein D containing ultrafiltration retentate is finally passed through a 0.2 µm membrane.

### Large Scale Preparation

The extraction from harvested cells and the purification of protein D was performed as follows. The harvested broth is cooled and directly passed twice through a high pressure homogenizer at a Pressure of around 800 bars.

In the first purification step the cell culture homogenate is diluted and applied to a cation exchange chromatography column (SP Sepharose Big beads). PD binds to the gel matrix by ionic interaction and is eluted by a step increase of the ionic strength of the elution buffer and filtrated.

In a second purification step impurities are retained on an anionic exchange matrix (Q Sepharose Fast Flow). PD does not bind onto the gel and can be collected in the flow through.

In both column chromatography steps fraction collection is monitored by OD. The flow through of the anionic exchange column chromatography containing the purified protein D is concentrated and diafiltrated by ultrafiltration.

The protein D containing ultrafiltration retentate is finally passed through a 0.2 µm membrane.

### Example 1 b: EXPRESSION OF PhtD

The PhtD protein is a member of the pneumococcal histidine-triad (Pht) protein family characterized by the presence of histidine-triads (HXXHXH motif). PhtD is a 838 aamolecule and carries 5 histidine triads (see Medlmmune WO00/37105 SEQ ID NO: 4 for amino acid sequence and SEQ ID NO: 5 for DNA sequence). PhtD also contains a proline-rich region in the middle (amino acid position 348-380). PhtD has a 20 aa-N-terminal signal sequence with a LXXC motif.

### Genetic construct

The gene sequence of the mature Medlmmune PhtD protein (from aa 21 to aa 838) was transferred recombinantly to *E. coli* using the in-house pTCMP14 vector carrying the pλ promoter. The *E. coli* host strain is AR58, which carries the cI857 thermosensitive repressor, allowing heat-induction of the promotor.

Polymerase chain reaction was realized to amplify the *phtD* gene from a Medlmmune plasmid (carrying the *phtD* gene from *Streptococcus pneumoniae* strain Norway 4 (serotype 4) - SEQ ID NO: 5 as described in WO 00/37105). Primers, specific for the *phtD* gene only, were used to amplify the *phtD* gene in two fragments. Primers carry either the *Nde*I and *Kpn*I or the *Kpn*I and *Xba*I restriction sites. These primers do not hybridize with any nucleotide from the vector but only with *phtD* specific gene sequences. An artificial ATG start codon was inserted using the first primer carrying the *Nde*I restriction site. The generated PCR products were then inserted into the pGEM-T cloning vector (Promega), and the DNA sequence was confirmed. Subcloning of the fragments in the TCMP14 expression vector was then realized using standard techniques and the vector was transformed into *AR58 E. coli.*

### PhtD Purification

PhtD purification is achieved as follows:
□ Growth of E.coli cells in the presence of Kanamycin: growth 30 hours at 30°C then induction for 18 hours at 39.5°C
□ Breakage of the E.coli cells from whole culture at OD ±115 in presence of EDTA 5 mM and PMSF 2 mM as protease inhibitors: Rannie, 2 passages, 1000 bars.
□ Antigen capture and cells debris removal on expanded bed mode Streamline Q XL chromatography at room temperature (20°C); the column is washed with NaCl 150 mM + Empigen 0.25% pH 6.5 and eluted with NaCl 400 mM + Empigen 0.25% in 25 mM potassium phosphate buffer pH 7.4.
□ Filtration on Sartobran 150 cartridge (0.45 + 0.2 µm)
□ Antigen binding on Zn⁺⁺ Chelating Sepharose FF IMAC chromatography at pH 7.4 in presence of 5 mM imidazole at 4°C; the column is washed with Imidazole 5 mM and Empigen 1% and eluted with 50 mM imidazole, both in 25 mM potassium phosphate buffer pH 8.0.
□ Weak anion exchange chromatography in positive mode on Fractogel EMD DEAE at pH 8.0 (25 mM potassium phosphate) at 4°C; the column is washed with 140 mM NaCl and eluted at 200 mM NaCl while contaminants (proteins and DNA) remain adsorbed on the exchanger.
□ Concentration and ultrafiltration with 2 mM Na/K phosphate pH 7.15 on 50 kDa membrane.
□ Sterilising filtration of the purified bulk on a Millipak-20 0.2 µm filter cartridge.

### Example 1c: EXPRESSION OF PNEUMOLYSIN

Pneumococcal pneumolysin was prepared and detoxified as described in WO2004/081515 and WO2006/032499.

### Example 2:

### Preparation of conjugates

It is well known in the art how to make purified pneumococcal polysaccharides. For the purposes of these examples the polysaccharides were made essentially as described in EP072513 or by closesly-related methods. Before conjugation the polysaccharides may be sized by microfluidisation as described below.

The activation and coupling conditions are specific for each polysaccharide. These are given in Table 1. Sized polysaccharide (except for PS5, 6B and 23F) was dissolved in NaCl 2M, NaCl 0.2M or in water for injection (WFI). The optimal polysaccharide concentration was evaluated for all the serotypes. All serotypes except serotype 18C were conjugated directly to the carrier protein as detailed below. Two alternative serotype 22F conjugates were made; one conjugated directly, one through an ADH linker.

From a 100 mg/ml stock solution in acetonitrile or acetonitrile/water 50%/50% solution, CDAP (CDAP/PS ratio 0.5-1.5 mg/mg PS) was added to the polysaccharide solution. 1.5 minute later, 0.2M-0.3M NaOH was added to obtain the specific activation pH. The activation of the polysaccharide was performed at this pH during 3 minutes at 25 °C. Purified protein (protein D, PhtD, pneumolysin or DT) (the quantity depends on the initial PS/carrier protein ratio) was added to the activated polysaccharide and the coupling reaction was performed at the specific pH for up to 2 hour (depending upon serotype) under pH regulation. In order to quench un-reacted cyanate ester groups, a 2M glycine solution was then added to the mixture. The pH was adjusted to the quenching pH (pH 9.0). The solution was stirred for 30 minutes at 25 °C and then overnight at 2-8 °C with continuous slow stirring.

### Preparation of 18C:

18C was linked to the carrier protein via a linker-Adipic acid dihydrazide (ADH) Polysaccharide serotype 18C was microfluidized before conjugation.

### Derivatization of tetanus toxoid with EDAC

For derivatization of the tetanus toxoid, purified TT was diluted at 25 mg/ml in 0.2M NaCl and the ADH spacer was added in order to reach a final concentration of 0.2M. When the dissolution of the spacer was complete, the pH was adjusted to 6.2. EDAC (1-ethyl-3-(3-dimethyl-aminopropyl) carbodiimide) was then added to reach a final concentration of 0.02M and the mixture was stirred for 1 hour under pH regulation. The reaction of condensation was stopped by increasing pH up to 9.0 for at least 30 minutes at 25°C. Derivatized TT was then diafiltrated (10 kDa CO membrane) in order to remove residual ADH and EDAC reagent.
TT_{AH} bulk was finally sterile filtered until coupling step and stored at -70°C.

### Chemical coupling of TT_{AH} to PS 18C

Details of the conjugation parameters can be found in Table 1. 2 grams of microfluidized PS were diluted at the defined concentration in water and adjusted to 2M NaCl by NaCl powder addition.

CDAP solution (100 mg/ml freshly prepared in 50/50 v/v acetonitrile/WFI) was added to reach the appropriate CDAP/PS ratio.

The pH was raised up to the activation pH 9.0 by the addition of 0.3M NaOH and was stabilised at this pH until addition of TT_{AH}.

After 3 minutes, derivatized TT_{AH} (20 mg/ml in 0.2 M NaCl) was added to reach a ratio TT_{AH} /PS of 2; the pH was regulated to the coupling pH 9.0. The solution was left one hour under pH regulation.

For quenching, a 2M glycine solution, was added to the mixture PS/TT_{AH}/CDAP.

The pH was adjusted to the quenching pH (pH 9.0).

The solution was stirred for 30 min at 25°C, and then left overnight at 2-8°C with continuous slow stirring.

### PS22F_{AH}-PhtD conjugate

In a second conjugation method for this saccharide (the first being the direct PS22-PhtD conjugation method shown in Table 1), 22F was linked to the carrier protein via a linker - Adipic acid dihydrazide (ADH). Polysaccharide serotype 22F was microfluidized before conjugation.

### PS 22F derivatization

Activation and coupling are performed at 25°C under continuous stirring in a temperature-controlled waterbath.

Microfluidized PS22F was diluted to obtain a final PS concentration of 6 mg/ml in 0.2M NaCl and the solution was adjusted at pH 6.05 ± 0.2 with 0.1 N HCl.

CDAP solution (100 mg/ml freshly prepared in acetonitrile/WFI, 50/50) was added to reach the appropriate CDAP/PS ratio (1.5/1 ww).

The pH was raised up to the activation pH 9.00 ±0.05 by the addition of 0.5M NaOH and was stabilised at this pH until addition of ADH.

After 3 minutes, ADH was added to reach the appropriate ADH/PS ratio (8.9/1 w/w); the pH was regulated to coupling pH 9.0. The solution was left for 1 hour under pH regulation.

The PS_{AH} derivative was concentrated and diafiltrated.

### Coupling

PhtD at 10 mg/ml in 0.2M NaCl was added to the PS22F_{AH} derivative in order to reach a PhtD/PS22F_{AH} ratio of 4/1 (w/w). The pH was adjusted to 5.0 ± 0.05 with HCl. The EDAC solution (20 mg/ml in 0.1 M Tris-HCl pH 7.5) was added manually in 10 min (250 µl / min) to reach 1 mg EDAC/mg PS22F_{AH}. The resulting solution was incubated for 150 min (though 60 mins was also used) at 25°C under stirring and pH regulation. The solution was neutralized by addition of 1 M Tris-HCl pH 7.5 (1/10 of the final volume) and let 30 min at 25°C.

Prior to the elution on Sephacryl S400HR, the conjugate was clarified using a 5µm Minisart filter.

The resulting conjugate has a final PhtD/PS ratio of 4.1 (w/w), a free PS content below 1% and an antigenicity (α-PS/α-PS) of 36.3% and anti-PhtD antigenicity of 7.4%.

### Purification of the conjugates:

The conjugates were purified by gel filtration using a Sephacryl S400HR gel filtration column equilibrated with 0.15M NaCl (S500HR for 18C) to remove small molecules (including DMAP) and unconjugated PS and protein. Based on the different molecular sizes of the reaction components, PS-PD, PS-TT, PS-PhtD, PS-pneumolysin or PS-DT conjugates are eluted first, followed by free PS, then by free PD or free DT and finally DMAP and other salts (NaCl, glycine).

Fractions containing conjugates are detected by UV_{280 nm}. Fractions are pooled according to their Kd, sterile filtered (0.22µm) and stored at +2-8°C. The PS/Protein ratios in the conjugate preparations were determined.

### Specific activation/coupling/guenching conditions of PS S. pneumoniae-Protein D/TT/DT/PhtD/Plyconjugates

Where "**µfluid**" appears in a row header, it indicates that the saccharide was sized by microfluidisation before conjugation. Sizes of saccharides following microfluidisation are given in table 2.

**Table 1 Specific activation/coupling/quenching conditions of PS S. pneumoniae-Protein D/TT/DT/PhtD/Plyconjugates**

| **Serotype** | **1 µfluid** | **4 µfluid** | **5** | **6A** | **6B** | **7F µfluid** |
|---|---|---|---|---|---|---|
| **PS conc.(mg/ml)** | 2.5 | 2.5 | 7.1 | 5.0 | 5.0 | 5.0 |
| **PS dissolution** | WFI | WFI | WFI | NaCl 2M | NaCl 2M | NaCl 2M |
| **PD conc.(mg/ml)** | 10.0 | 10.0 | 5.0 | 5.0 | 5.0 | 10.0 |
| **Initial PD/PS Ratio (w/w)** | 1.5/1 | 1.5/1 | 1/1 | 1/1 | 1.1/1 | 1.2/1 |
| **CDAP conc.** | 0.50 | 0.50 | 0.79 | 0.83 | 0.83 | 0.75 |
| **(mg/mg PS)** | | | | | | |
| **pHₐ=pH_{c}=pH_{q}** | 9.0/9.0/9.0 | 9.5/9.5/9.0 | 9.0/9.0/9.0 | 9.5/9.5/9.0 | 9.5/9.5/9.0 | 9.5/9.5/9.0 |

**Note: pHa,c,q corresponds to the pH for activation, coupling and quenching, respectively**

| **Serotype** | **9V µfluid** | **14 µfluid** | **18C µfluid** | **19A µfluid** | **19F µfluid** | **22F µfluid** | **23F** |
|---|---|---|---|---|---|---|---|
| **PS conc.(mg/ml)** | 5.0 | 5.0 | 4.5 | 15.0 | 9.0 | 6.0 | 2.38 |
| **PS dissolution** | NaCl 2M | NaCl 2M | NaCl 2M | NaCl 2M | NaCl 2M | NaCl 0.2M | NaCl 2M |
| **Carrier protein conc.(mg/ml)** | 10.0 | 10.0 | 20.0 (TT) | 10.0 (Ply) | 20.0 (DT) | 10.0 (PhtD) | 5.0 |
| **Initial carrier protein/PS Ratio (w/w)** | 1.2/1 | 1.2/1 | 2/1 | 2.5/1 | 1.5/1 | 3/1 | 1/1 |
| **CDAP conc. (mg/mg PS)** | 0.50 | 0.75 | 0.75 | 1.5 | 1.5 | 1.5 | 0.79 |
| **pHₐ=pH_{c}=pH_{q}** | 9.5/9.5/9.0 | 9.5/9.5/9.0 | 9.0/9.0/9.0 | 9.0/9.0/9.0 | 9.0/9.0/9.0 | 9.0/9.0/9.0 | 9.5/9.5/9.0 |

### Characterisation:

Each conjugate was characterised and met the specifications described in Table 2. The polysaccharide content (µg/ml) was measured by the Resorcinol test and the protein content (µg/ml) by the Lowry test. The final PS/PD ratio (w/w) is determined by the ratio of the concentrations.

### Free polysaccharide content (%):

The free polysaccharide content of conjugates kept at 4°C or stored 7 days at 37°C was determined on the supernatant obtained after incubation with α-carrier protein antibodies and saturated ammonium sulfate, followed by a centrifugation.

An α-PS/α-PS ELISA was used for the quantification of free polysaccharide in the supernatant. The absence of conjugate was also controlled by an α-carrier protein/α-PS ELISA.

### Antigenicity:

The antigenicity on the same conjugates was analyzed in a sandwich-type ELISA wherein the capture and the detection of antibodies were α-PS and α-Protein respectively.

### Free protein content (%):

Unconjugated carrier protein can be separated from the conjugate during the purification step. The content of free residual protein was determined using size exclusion chromatography (TSK 5000-PWXL) followed by UV detection (214 nm). The elution conditions allowed separating the free carrier protein and the conjugate. Free protein content in conjugate bulks was then determined versus a calibration curve (from 0 to 50 µg/ml of carrier protein). Free carrier protein in % was obtained as follows: % free carrier = (free carrier (µg/ml)/(Total concentration of corresponding carrier protein measured by Lowry (µg/ml)* 100%).

### Stability:

Molecular weight distribution (Kₐᵥ) and stability was measured on a HPLC-SEC gel filtration (TSK 5000-PWXL) for conjugates kept at 4°C and stored for 7 days at 37°C.

The 10/11/13/14-valent characterization is given in Table 2 (see comment thereunder).

The protein conjugates can be adsorbed onto aluminium phosphate and pooled to form the final vaccine.

### Conclusion:

Immunogenic conjugates have been produced, that have since been shown to be components of a promising vaccine.

**TABLE 2 - characteristics of the conjugates**

| Conjugates | PS size (Dax10³) | Carrier/PS Ratio | Free PS (Elisa) | Free Carrier | PS Antigenicity (Elisa) | Conj Size (kDa) |
|---|---|---|---|---|---|---|
| PS1-PD | 349-382* | 1.5-1.6 | 1.0%-1.2% | 3.9%-4.8% | 87%-95% | 1499-1715 |
| PS4-PD | 93-100* | 1.5-1.6 | 4.7-6.5% | 3.2%-4.0% | 90%-96% | 1303-1606 |
| PS5-PD*** | 367-443 | 0.80 | 8.7-11.2% | 2.2%-3.8% | 93%-108% | 1998-2352 |
| PS6A-PD | 1100-1540 | 0.61 | 4.5% | Not done | 45.9% | Not done |
| PS6B-PD*** | 1069-1391 | 0.7-0.8 | 1.3-1.6% | <2.0% | 68%-75% | 4778-5235 |
| PS7F-PD | 255-264* | 1.1-1.2 | <1% | <1.4% | 58% | 3907-4452 |
| PS9V-PD | 258-280* | 1.3-1.5 | <1% | <1.3% | 67%-69% | 9073-9572 |
| PS14-PD | 232-241* | 1.4 | <1% | <1.5% | 70% | 3430-3779 |
| PS18C-TT" | 89-97* | 2.2-2.4 | 1.5-2.2% | <4% | 46%-56% | 5464-6133 |
| PS19A-Ply* | 151 | 3.2 | <1% | | 29% | |
| PS19F-DT | 133-143* | 1.4-1.5 | 4.1%-5.9% | <1.2%-<1.3% | 82%-88% | 2059-2335 |
| PS22F-PhtD* | 159-167 | 2.17 | 5.8 | Not done | 37% | Not done |
| PS22F-AHPhtD* | 159-167 | 3.66-4.34 | <1% | Not done | 28-31% | Not done |
| PS23F-PD*** | 914-980 | 0.5 | 1.4-1.9% | 3.7%-4.9% | 137%-154% | 2933-3152 |

| | | | | | | |
|---|---|---|---|---|---|---|
| * PS size following microfluidization of the native PS | | | | | | |

A 10 valent vaccine was made by mixing serotype 1, 4, 5, 6B, 7F, 9V, 14, 18C, 19F and 23F conjugates (e.g. at a dose of 1, 3, 1, 1, 1, 1, 1, 3, 3, 1 µg of saccharide, respectively per human dose). An 11 valent vaccine was made by further adding the serotype 3 conjugate from Table 5 (e.g. at 1 µg of saccharide per human dose). A 13 valent vaccine was made by further adding the serotypes 19A and 22F conjugates above (with 22F either directly linked to PhtD, or alternatively through an ADH linker) [e.g. at a dose of 3 µg each of saccharide per human dose]. A 14 valent vaccine may be made by further adding the serotype 6A conjugate above [e.g. at a dose of 1 µg of saccharide per human dose.

### Example 3: Evidence that inclusion of Haemphilus influenzae protein D in an immunogenic composition of the invention can provide improved protection against acute otitis media (AOM).

### Study design.

The study used an 11Pn-PD vaccine - comprising serotypes 1, 3, 4, 5, 6B, 7F, 9V, 14, 18C, 19F and 23F each conjugated to protein D from *H*. influenzae (refer to Table 5 in Example 4). Subjects were randomized into two groups to receive four doses of either the 11 Pn-PD vaccine or *Havrix* at approximately 3, 4, 5 and 12-15 months of age. All subjects received GSK Biologicals' *Infanrix- hexa* (DTPa-HBV-IPV/Hib) vaccine concomitantly at 3, 4 and 5 months of age. *Infanrix- hexa* is a combination of *Pediarix* and Hib mixed before administration. Efficacy follow-up for the "According-to-Protocol" analysis started 2 weeks after administration of the third vaccine dose and continued until 24-27 months of age. Nasopharyngeal carriage of *S*. *pneumoniae* and *H. influenzae* was evaluated in a selected subset of subjects.

Parents were advised to consult the investigator if their child was sick, had ear pain, spontaneous perforation of the tympanic membrane or spontaneous ear discharge. If the investigator suspected an episode of AOM, the child was immediately referred to an Ear, Nose and Throat (ENT) specialist for confirmation of the diagnosis.

A clinical diagnosis of AOM was based on either the visual appearance of the tympanic membrane (i.e. redness, bulging, loss of light reflex) or the presence of middle ear fluid effusion (as demonstrated by simple or pneumatic otoscopy or by microscopy). In addition, at least two of the following signs or symptoms had to be present: ear pain, ear discharge, hearing loss, fever, lethargy, irritability, anorexia, vomiting, or diarrhea. If the ENT specialist confirmed the clinical diagnosis, a specimen of middle ear fluid was collected by tympanocentesis for bacteriological testing.

For subjects with repeated sick visits, a new AOM episode was considered to have started if more than 30 days had elapsed since the beginning of the previous episode. In addition, an AOM episode was considered to be a new bacterial episode if the isolated bacterium/serotype differed from the previous isolate whatever the interval between the two consecutive episodes.

### Trial results

A total of 4968 infants were enrolled, 2489 in the 11Pn-PD group and 2479 in the control group. There were no major differences in the demographic characteristics or risk factors between the two groups.

### Clinical episodes and AOM case definition

During the per protocol follow-up period, a total of 333 episodes of clinical AOM were recorded in the 11Pn-PD group and 499 in the control group.

Table 3 presents the protective efficacy of the 11Pn-PD vaccine and both 7-valent vaccines previously tested in Finland (Eskola et al N Engl J Med 2001; 344: 403 - 409 and Kilpi et alClin Infect Dis 2003 37:1155-64) against any episode of AOM and AOM caused by different pneumococcal serotypes, *H. influenzae, NTHi* and *M*. *catarrhalis.*

Statistically significant and clinically relevant reduction by 33.6% of the overall AOM disease burden was achieved with 11 Pn-PD, irrespective of the etiology (table 3).

The overall efficacy against AOM episodes due to any of the 11 pneumococcal serotypes contained in the 11 Pn-PD vaccine was 57.6% (table 3).

Another important finding in the current study is the 35.6% protection provided by the 11 Pn-PD vaccine against AOM caused by *H. influenzae* (and specifically 35.3% protection provided by NTHi). This finding is of major clinical significance, given the increased importance of *H. influenzae* as a major cause of AOM in the pneumococcal conjugate vaccine era. In line with the protection provided against AOM, the 11Pn-PD vaccine also reduced nasopharyngeal carriage of *H. influenzae* following the booster dose in the second year of life. These findings are in contrast with previous observations in Finland where, for both 7-valent pneumococcal conjugate vaccines, an increase in AOM episodes due to *H. influenzae* was observed, (Eskola *et al* and Kilpi *et al*) as evidence of etiological replacement.

A clear correlation between protection against AOM episodes due to Hi and antibody levels against the carrier Protein D could not be established, as post-primary anti-PD IgG antibody concentrations in 11Pn-PD vaccinees, that remained Hi AOM episode-free, were essentially the same as post-primary anti-PD IgG antibody levels measured in 11Pn-PD vaccinees that developed at least one Hi AOM episode during the efficacy follow-up period. However, although no correlation could be established between the biological impact of the vaccine and the post-primary IgG anti-PD immunogenicity, it is reasonable to assume that the PD carrier protein, which is highly conserved among *H. influenzae* strains, has contributed to a large extent in the induction of the protection against Hi.

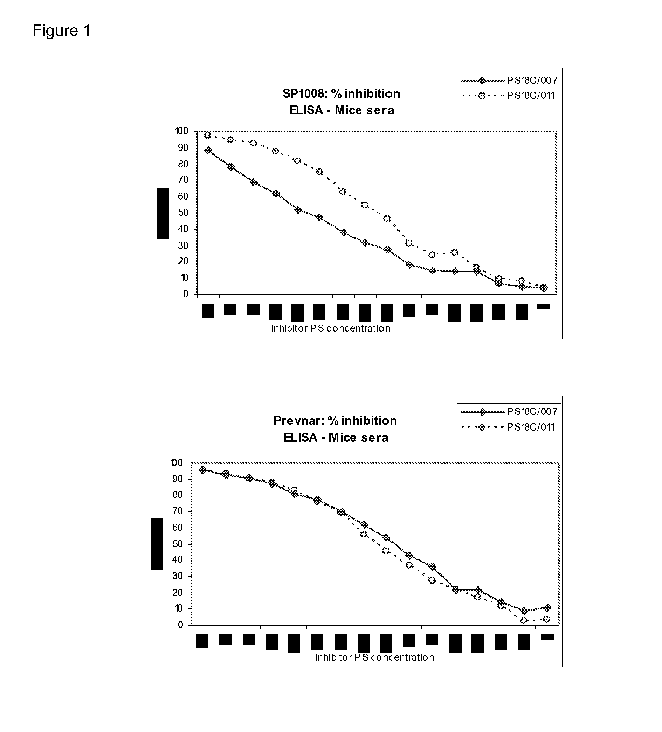

The effect on AOM disease was accompanied by an effect on nasopharyngeal carriage that was of similar magnitude for vaccine serotype pneumococci and *H. influenzae* (Figure 1). This reduction of the nasopharyngeal carriage of *H. influenzae* in the PD-conjugate vaccinees supports the hypothesis of a direct protective effect of the PD-conjugate vaccine against *H. influenzae,* even if the protective efficacy could not be correlated to the anti-PD IgG immune responses as measured by ELISA.

In a following experiment a chinchilla otitis media model was used with serum pools from infants immunised with the 11 valent formulation of this example or with the 10 valent vaccine of Example 2 (see also Table 1 and 2 and comments thereunder). Both pools induce a significant reduction of the percentage of animals with otitis media versus the pre-immune serum pool. There is no significant difference beteen the 10 and 11 valent immune pools. This demonstrates that both vaccines have a similar potential to induce protection against otitis media caused by non typeable *H. influenzae* in this model.

**Table 3**

| **Type of AOM episode** | **11Pn-PD** | | | | | **Prevnar in FinOM (Eskola et al)** | | | | | **7v-OMP in FinOM ^{(Kilip et al)}** | | | | |
|---|---|---|---|---|---|---|---|---|---|---|---|---|---|---|---|
| | **n** | | **VE** | | | **n** | | **VE** | | | **n** | | **VE** | | |
| | **11Pn-PD** | **Control** | % | **95%Cl** | | **7v-CRM** | **Control** | % | **95%Cl** | | **7v-OMP** | **Control** | % | **95%Cl** | |
| | | | | **LL** | **UL** | | | | **LL** | **UL** | | | | **LL** | **UL** |
| N | 2455 | 2452 | | | | 786 | 794 | | | | 805 | 794 | | | |
| Any AOM | 333 | 499 | **33.6** | 20.8 | 44.3 | 1251 | 1345 | **6** | -4 | 16 | 1364 | 1345 | **-1** | -12 | 10 |
| Any AOM with MEF | 322 | 474 | **32.4** | 19.0 | 43.6 | 1177 | 1267 | **7** | -5 | 17 | 1279 | 1267 | **0** | -12 | 10 |
| Culture confirmed pneumococcus | 92 | 189 | **51.5** | 36.8 | 62.9 | 271 | 414 | **34** | 21 | 45 | 314 | 414 | **25** | 11 | 37 |
| Vaccine pneumococcal serotypes(*) | 60 | 141 | **57.6** | 41.4 | 69.3 | 107 | 250 | **57** | 44 | 67 | 110 | 250 | **56** | 44 | 66 |
| Other bacterial pathogens | | | | | | | | | | | | | | | |
| *H. influenzae* | 44 | 68 | **35.6** | 3.8 | 57.0 | 315 | 287 | **-11** | -34 | 8 | 315 | 287 | **-9** | -32 | 10 |
| Non-typeable *H*. *influenzae* (NTHi) | 41 | 63 | **35.3** | 1.8 | 57.4 | NP | NP | **NP** | NP | NP | NP | NP | **NC** | NP | NP |
| *M*. *catarrhalis* | 31 | 34 | **9.4** | -52.5 | 46.1 | 379 | 381 | **-1** | -19 | 15 | 444 | 381 | **-16** | -36 | 2 |

| | | | | | | | | | | | | | | | |
|---|---|---|---|---|---|---|---|---|---|---|---|---|---|---|---|
| NP = Not published, N = number of subjects in ATP efficacy cohort, n = number of episodes *Vaccine pneumococcal serotypes for 11Pn-PD = 11 serotypes, for Prevnar and 7v-OMP = 7 serotypes MEF = Middle ear fluid | | | | | | | | | | | | | | | |

### Example 4:

### Selection of carrier protein for serotype 19F

### ELISA assay used

The 22F inhibition ELISA method was essentially based on an assay proposed in 2001 by Concepcion and Frasch and was reported by Henckaerts et al., 2006, Clinical and Vaccine Immunology 13:356-360. Briefly, purified pneumococcal polysaccharides were mixed with methylated human serum albumin and adsorbed onto Nunc Maxisorp™ (Roskilde, DK) high binding microtitre plates overnight at 4°C. The plates were blocked with 10% fetal bovine serum (FBS) in PBS for 1 hour at room temperature with agitation. Serum samples were diluted in PBS containing 10% FBS, 10 µg/mL cell-wall polysaccharide (SSI) and 2 µg/mL of pneumococcal polysaccharide of serotype 22F (ATCC), and further diluted on the microtitre plates with the same buffer. An internal reference calibrated against the standard serum 89-SF using the serotype-specific IgG concentrations in 89-SF was treated in the same way and included on every plate. After washing, the bound antibodies were detected using peroxidase-conjugated anti-human IgG monoclonal antibody (Stratech Scientific Ltd., Soham, UK) diluted in 10% FBS (in PBS), and incubated for 1 hour at room temperature with agitation. The color was developed using ready-to-use single component tetramethylbenzidine peroxidase enzyme immunoassay substrate kit (BioRad, Hercules, CA, US) in the dark at room temperature. The reaction was stopped with H2SO4 0.18 M, and the optical density was read at 450 nm. Serotype-specific IgG concentrations (in µg/mL) in the samples were calculated by referencing optical density points within defined limits to the internal reference serum curve, which was modelized by a 4-parameter logistic log equation calculated with SoftMax Pro™ (Molecular Devices, Sunnyvale, CA) software. The cut-off for the ELISA was 0.05 µg/mL IgG for all serotypes taking into account the limit of detection and the limit of quantification.

### Opsonophagocytosis assay

At the WHO consultation meeting in June 2003, it was recommended to use an OPA assay as set out in Romero-Steiner et al Clin Diagn Lab Immunol 2003 10 (6): pp1019-1024. This protocol was used to test the OPA activity of the serotypes in the following tests.

### Preparation of conjugates

In studies 11 Pn-PD&Di-001 and 11 Pn-PD&Di-007, three 11-valent vaccine formulations (Table 4) were included in which 3µg of the 19F polysaccharide was conjugated to diphtheria toxoid (19F-DT) instead of 1µg polysaccharide conjugated to protein D (19F-PD). Conjugation parameters for the studies 11Pn-PD, 11 Pn-PD&Di-001 and 11Pn-PD&Di-007 are disclosed in Tables 5, 6 and 7 respectively.

Anti-pneumococcal antibody responses and OPA activity against serotype 19F one month following primary vaccination with these 19F-DT formulations are shown in Table 8 and 9 respectively.

Table 10 shows 22F-ELISA antibody concentrations and percentages of subjects reaching the 0.2 µg/mL threshold before and after 23-valent plain polysaccharide booster vaccination.

The opsonophagocytic activity was shown to be clearly improved for antibodies induced with these 19F-DT formulations as demonstrated by higher seropositivity rates (opsonophagocytic titers ≥ 1:8) and OPA GMTs one month following primary vaccination (Table 9). One month after 23-valent plain polysaccharide booster vaccination, opsonophagocytic activity of 19F antibodies remained significantly better for children primed with 19F-DT formulations (Table 11).

Table 12 presents immunogenicity data following a 11Pn-PD booster dose in toddlers previously primed with 19F-DT or 19F-PD conjugates compared to a 4^{th} consecutive dose of *Prevnar*®*.* Given the breakthrough cases reported after the introduction of *Prevnar*® in the US, the improved opsonophagocytic activity against serotype 19F when conjugated to the DT carrier protein may be an advantage for the candidate vaccine.

Table 13 provides ELISA and OPA data for the 19F-DT conjugate with respect to the cross-reactive serotype 19A. It was found that 19F-DT induces low but significant OPA activity against 19A.

**Table4 Pneumococcal conjugate vaccine formulations used in clinical studies.**

| **Formulation** | **Pneumococcal serotype µg/carrier protein** | | | | | | | | | | | **Al³⁺ mg** |
|---|---|---|---|---|---|---|---|---|---|---|---|---|
| | **1** | **3** | **4** | **5** | **6B** | **7F** | **9V** | **14** | **18C** | **19F** | **23F** | |
| 11Pn-PD | 1/PD | 1/PD | 1/PD | 1/PD | 1/PD | 1/PD | 1/PD | 1/PD | 1/PD | 1/PD | 1/PD | <0.8 |
| 19F-DT Form 1 | 3/PD | 3/PD | 3/PD | 3/PD | 10/DT | 3/PD | 3/PD | 3/PD | 3/PD | 3/DT | 5/DT | ≤0.35 |
| 19F-DT Form 2 | 3/PD | 2/PD | 2/PD | 3/PD | 5/DT | 3/PD | 2/PD | 2/PD | 2/PD | 3/DT | 5/DT | ≤0.35 |
| 19F-DT Form 3 | 3/PD | 3/PD | 3/PD | 3/PD | 3/PD | 3/PD | 3/PD | 3/PD | 3/PD | 3/DT | 3/PD | =0.5 |

**Table 5 Specific activation/coupling/quenching conditions of PS S.Pneumoniae-Protein D/TT/DTconujgates**

| Serotype | 1 | 3 | 4 | 5 | 6B | 7F |
|---|---|---|---|---|---|---|
| | Native | µfluid | Native | Native | Native | Native |
| PS conc.(mp/ml) | 1.5 | 2 | 2.0 | 7.5 | 5.5 | 3.0 |
| PS dissolution | NaCl 150mM | NaCl 2M | WFI | WFI | NaCl 2M | NaCl 2M |
| PD conc.(mp/ml) | 5.0 | 5.0 | 5.0 | 5.0 | 5.0 | 5.0 |
| Initial PS/PD Ratio (w/w) | 1/0.7 | 1/1 | 1/1 | 1/1 | 1/1 | 1/1 |
| CDAP conc. (mg/mg PS) | 0.75 | 0.75 | 0.75 | 0.75 | 0.75 | 0.75 |
| pHₐ=pH_{c}=pH_{q} | 9.0/9.0/9.0 | 9.5/9.5/9.0 | 8.8/8.8/9.0 | 9.0/9.0/9.0 | 9.5/9.5/9.0 | 9.0/9.0/9.0 |
| Coupling time | 60 mins | 60 mins | 45 mins | 40 mins | 60 mins | 60 mins |

| Serotype | 9V | 14 | 18C | 19F | 23F |
|---|---|---|---|---|---|
| | Native | Native | Native | Native | Native |
| PS conc.(mp/ml) | 1.75 | 2.5 | 1.75 | 4.0 | 2.5 |
| PS dissolution | NaCl 2M | NaCl 2M | WFI | NaCl 2M | NaCl 2M |
| PD conc.(mp/ml) | 5.0 | 5.0 | 5.0 | 5.0 | 5.0 |
| Initial PS/PD Ratio (w/w) | 1/0.75 | 1/0.75 | 1/1.2 | 1/1 | 1/1 |
| CDAP conc. (mg/mg PS) | 0.75 | 0.75 | 0.75 | 0.75 | 0.75 |
| pHₐ=pH_{c}=pH_{q} | 8.5/8.5/9.0 | 9.0/9.0/9.0 | 9.0/9.0/9.0 | 9.5/9.5/9.0 | 9.5/9.5/9.0 |
| Coupling time | 60 mins | 60 mins | 45 mins | 30 mins | 60 mins |

**Table 6 Specific activation/coupling/quenching conditions of PS S.Pneumoniae-Protein D/DTconjugates for the 11 Pn-PD&Di-001 study**

| Serotype | 1 | 3 | 4 | 5 | 6B | 7F |
|---|---|---|---|---|---|---|
| | µfluid | µfluid | µfluid | µfluid | µfluid | Native |
| PS conc.(mg/ml) | 4 | 2.0 | 2.5 | 7.5 | 10 | 3.0 |
| PS dissolution | NaCl 2M | NaCl 2M | NaCl 2M | NaCl 2M | NaCl 2M | NaCl 2M |
| PD conc.(mg/ml) | 10.0 | 5.0 | 5.0 | 5.0 NaCl 2M | 20 (DT) NaCl 2M | 5.0 |
| Initial PD/PS Ratio (w/w) | 1.2/1 | 1/1 | 1/1 | 1/1 | 1.5/1 | 1/1 |
| CDAP conc. (mglmg PS) | 1.50 | 0.75 | 1.5 | 2 | 1.5 | 0.75 |
| pHₐ=pH_{c}=pH_{q} | 9.0/9.0/9.0 | 9.5/9.5/9.0 | 9.5/9.5/9.0 | 9.0/9.0/9.0 | 9.5/9.5/9.0 | 9/9/9 |
| Coupling time | 60 mins | 60 mins | 60 mins | 60 mins | 60 mins | 60 mins |

| Serotype | 9V | 14 | 18C | 19F | 23F |
|---|---|---|---|---|---|
| | Native | Native | µfluid | µfluid | µfluid |
| PS conc.(mg/ml) | 1.75 | 2.5 | 5.0 | 9.0 | 10 |
| PS dissolution | NaCl 2M | NaCl 2M | NaCl 2M | NaCl 2M | NaCl 2M |
| Carrier protein conc.(mg/ml) | 5.0 | 5.0 | 5.0 | 20 (DT) | 10 (DT) |
| Initial carrier protein/PS Ratio (w/w) | 0.75/1 | 0.75/1 | 1.2/1 | 1.5/1 | 1.5/1 |
| CDAP conc. (mg/mg PS) | 0.75 | 0.75 | 1.5 | 1.5 | 0.75 |
| pHₐ=pH_{c}=pH_{q} | 8.5/8.5/9.0 | 9.0/9.0/9.0 | 9.0/9.0/9.0 | 9.0/9.0/9.0 | 9.5/9.5/9.0 |
| Coupling time | 60 mins | 60 mins | 30 mins | 60 mins | 60 mins |

**Table 7 Specific activation/coupling/quenching conditions of PS S.Pneumoniae-Protein D/DTconjugates for the 11Pn-PD&Di-007 study**

| Serotype | 1 | 3 | 4 | 5 | 6B | 7F |
|---|---|---|---|---|---|---|
| | Native | µfluid | Native | Native | Native | µfluid |
| PS conc.(mg/ml) | 1.5 | 2.0 | 2 | 7.5 | 5.5 | 5.0 |
| PS dissolution | NaCl | NaCl 2M | WFI | WFI | NaCl 2M | NaCl 2M |
| | 150 mM | | | | | |
| PD conc.(mg/ml) | 5.0 | 5.0 | 5.0 | 5.0 | 5 | 10 |
| Initial PD/PS Ratio (w/w) | 0.7/1 | 1/1 | 1 | 1/1 | 1/1 | 1.2/1 |
| CDAP conc. (mg/mg PS) | 0.75 | 0.75 | 0.75 | 0.75 | 0.75 | 0.75 |
| pHₐ=pH_{c}=pH_{q} | 9.0/9.0/9.0 | 9.5/9.5/9.0 | 8.8/8.8/9.0 | 9.0/9.0/9.0 | 9.5/9.5/9.0 | 9.5./9.5/9 |
| Coupling time | 60 mins | 60 mins | 45 mins | 40 mins | 60 mins | 60 mins |

| Serotype | 9V | 14 | 18C | 19F | 19F | 23F |
|---|---|---|---|---|---|---|
| | µfluid | µfluid | Native | µfluid | µfluid | µfluid |
| PS conc.(mg/ml) | 5.0 | 5.0 | 1.75 | 9.0 | 10.0 | 9.5 |
| PS dissolution | NaCl 2M | NaCl 2M | WFI | NaCl 2M | NaCl 2M | NaCl 2M |
| Carrier protein conc.(mg/ml) | 10 | 10.0 | 5.0 | 20 (DT) | 5.0 (PD) | 10 |
| Initial carrier protein/P S Ratio (w/w) | 1.2/1 | 1.2/1 | 1.2/1 | 1.5/1 | 1.2/1 | 1/1 |
| CDAP conc. (mg/mg PS) | 0.5 | 0.75 | 0.75 | 1.5 | 0.75 | 0.75 |
| pHₐ=pH_{c}=pH_{q} | 9.5/9.5/9.0 | 9.5/9.5/9.0 | 9.0/9.0/9.0 | 9.0/9.0/9.0 | 9.0/9.0/9.0 | 9.5/9.5/9.0 |
| Coupling time | 60 mins | 60 mins | 45 mins | 120 mins | 120 mins | 60 mins |

**Table 8 Percentage of subjects with 19F antibody concentrations 0.20 µg/mL and 19F antibody Geometric mean antibody concentrations (GMCs with 95% Cl; µg/mL) one month following 1µg 19F-PD, 3µg 19F-DT or Prevnar (2µg 19F-CRM) primary vaccination (Total cohort)**

| **Group** | **11Pn-PD&Di-001 (22F-ELISA)** | | | **11Pn-PD&Di-007 (22F-ELISA)** | | |
|---|---|---|---|---|---|---|
| | **N** | **% ≥ 0.20 µg/mL** | **GMC (µg/mL)** | **N** | **% ≥ 0.20 µg/mL** | **GMC (µg/mL)** |
| | | (95% Cl) | (95% Cl) | | (95% Cl) | (95% Cl) |
| 11Pn-PD | 152 | **98.7** | **1.93** | 50 | **100** | **2.78** |
| | | (95.3-99.8) | (1.67-2.22) | | (92.9-100) | (2.31-3.36) |
| 19F-DT Form 1^{Γ} | 146 | **99.3** (96.2-100) | **2.88** (2.45-3.38) | - | - | - |
| 19F-DT Form 2^{Γ} | 150 | **96.0** (91.5-98.5) | **2.43** (2.01-2.94) | - | - | - |
| 19F-DT Form 3^{Γ} | - | - | - | 50 | **96.0** (86.3-99.5) | **3.70** (2.58-5.30) |
| *Prevnar* | 148 | **98.6** (95.2-99.8) | **2.98** (2.60-3.41) | 41 | **97.6** (87.1-99.9) | **2.91** (2.15-3.94) |

| | | | | | | |
|---|---|---|---|---|---|---|
| ^{Γ} The composition of the different formulations is provided in table 4. | | | | | | |

**Table 9 Percentage of subjects with 19F OPA titer ≥ 1:8 and 19F OPA GMTs one month following primary vaccination with 1µg 19F-PD, 3µg 19F-DT or Prevnar (2µg 19F-CRM) (Total cohort)**

| **Group** | **11Pn-PD&Di-001** | | | **11Pn-PD&Di-007** | | |
|---|---|---|---|---|---|---|
| | **N** | **≥1:8** (95% Cl) | **GMT** (95% Cl) | **N** | **≥1:8** (95% Cl) | **GMT** (95% Cl) |
| 11Pn-PD | 136 | **84.6** (77.4-90.2) | **77.8** (58.1-104.4) | 46 | **95.7** (85.2-99.5) | **167.8** (118.1-238.6) |
| 19F-DT Form 1^{Γ} | 137 | **95.6** (90.7-98.4) | **263.2** (209.4-330.7) | - | - | - |
| 19F-DT Form 2^{Γ} | 139 | **92.1** (86.3-96.0) | **218.9** (166.5-287.9) | - | - | - |
| 19F-DT Form 3^{Γ} | - | - | - | 49 | **91.8** (80.4-97.7) | **403.1** (225.7-719.9) |
| *Prevnar* | 131 | **86.3** (79.2-91.6) | **82.6** (61.1-111.6) | 38 | **81.6** (65.7-92.3) | **65.0** (37.7-112.2) |

| | | | | | | |
|---|---|---|---|---|---|---|
| ^{Γ} The composition of the different formulations is provided in Table 4. | | | | | | |

**Table 10 Percentage of subjects with 19F antibody concentration ≥ 0.20 µg/mL and 19F antibody GMCs (µg/mL) prior to and one month following 23-valent plain polysaccharide booster in children primed with 1µg 19F-PD, 3µg 19F-DT or Prevnar (2µg 19F-CRM) (Total cohort)**

| **Primary group** | **11Pn-PD&Di-002 (22F ELISA)** | | | | | |
|---|---|---|---|---|---|---|
| | **Prior to booster vaccination** | | | **One month post 23-valent PS booster** | | |
| | **N** | **% ≥ 0.20 µg/mL** | **GMC (µg/ml)** | **N** | **% ≥ 0.20 µg/mL** | **GMC (µg/ml)** |
| | | (95% Cl) | (95% Cl) | | (95% Cl) | (95% Cl) |
| 11Pn-PD | 70 | **77.1** | **0.67** | 67 | **94.0** | **11.50** |
| | | (65.6-86.3) | (0.45-0.98) | | (85.4-98.3) | (7.76-17.03) |
| 19F-DT Form 1^{Γ} | 68 | **91.2** | **0.71** | 69 | **98.6** | **14.50** |
| | | (81.8-96.7) | (0.54-0.94) | | (92.2-100) | (10.47-20.07) |
| 19F-DT Form 2^{Γ} | 74 | **81.1** | **0.59** | 72 | **95.8** | **9.90** |
| | | (70.3-89.3) | (0.43-0.80) | | (88.3-99.1) | (6.74-14.54) |
| *Prevnar* | 65 | **64.6** | **0.40** | 67 | **100** | **9.40** |
| | | (51.8-76.1) | (0.27-0.60) | | (94.6-100) | (6.95-12.71) |

| | | | | | | |
|---|---|---|---|---|---|---|
| ^{Γ} The composition of the different formulations is provided in Table 4. | | | | | | |

**Table 11 Percentage of subjects with 19F OPA titer ≥ 1:8 and 19F OPA GMTs prior to and one month following 23-valent plain polysaccharide booster in children primed with 1µg 19F-PD, 3µg 19F-DT or Prevnar (2µg 19F-CRM) (Total cohort)**

| **Primary group** | **11Pn-PD&Di-002** | | | | | |
|---|---|---|---|---|---|---|
| | **Prior to booster vaccination** | | | **One month post 23-valent PS booster** | | |
| | **N** | **% ≥ 1:8** | **GMT** | **N** | **% ≥ 1:8** | **GMT** |
| | | (95% Cl) | (95% Cl) | | (95% Cl) | (95% Cl) |
| 11Pn-PD | 29 | **27.6** | **10.9** | 28 | **82.1** | **408.0** |
| | | (12.7-47.2) | (5.0-23.7) | | (63.1-93.9) | (157.3-1058.3) |
| 19F-DT Form 1^{Γ} | 19 | **47.4** | **18.1** | 18 | **94.4** | **1063.8** |
| | | (24.4-71.1) | (7.2-45.7) | | (72.7-99.9) | (386.6-2927.5) |
| 19F-DT Form 2^{Γ} | 27 | **33.3** | **8.5** | 28 | **100** | **957.6** |
| | | (16.5-54.0) | (4.7-15.3) | | (87.7-100) | (552.8-1659.0) |
| *Prevnar* | 24 | **12.5** | **8.1** | 23 | **82.6** | **380.9** |
| | | (2.7-32.4) | (3.4-19.6) | | (61.2-95.0) | (133.2-1089.5) |

| | | | | | | |
|---|---|---|---|---|---|---|
| ^{Γ}The composition of the different formulations is provided in Table 4. | | | | | | |

**Table 12 Percentage of subjects with antibody concentrations ≥ 0.2 µg/mL, OPA ≥ 1:8 and GMCs/GMTs against 19F pneumococci one month following 11Pn-PD or Prevnar booster in children primed with 1µg 19F-PD, 3µg 19F-DT or Prevnar (2µg 19F-CRM) (Total cohort)**

| **Primary group** | **11Pn-PD&Di-002** | | | | | |
|---|---|---|---|---|---|---|
| | **22F-ELISA assay** | | | **OPA assay** | | |
| | **N** | **% ≥ 0.20 µg/mL** | **GMC (µg/ml)** | **N** | **% ≥ 1:8** | **GMT** |
| | | (95% Cl) | (95% Cl) | | (95% Cl) | (95% Cl) |
| 11Pn-PD | 70 | **100** | **4.52** | 21 | **100** | **255.6** |
| | | (94.9-100) | (3.7-5.5) | | (83.9-100) | (135.5-481.9) |
| 19F-DT Form 1^{Γ} | 66 | **98.5** | **3.45** | 23 | **95.7** | **374.0** |
| | | (91.8-100) | (2.8-4.3) | | (78.1-99.9) | (192.6-726.2) |
| 19F-DT Form 2^{Γ} | 70 | **98.6** | **3.80** | 29 | **96.6** | **249.1** |
| | | (92.3-100) | (2.9-4.9) | | (82.2-99.9) | (144.7-428.7) |
| *Prevnar* | 69 | **97.1** | **2.56** | 31 | **96.8** | **528.7** |
| | | (89.9-99.6) | (2.0-3.3) | | (83.3-99.9) | (319.4-875.2) |

| | | | | | | |
|---|---|---|---|---|---|---|
| ^{Γ} The composition of the different formulations is provided in Table 4. | | | | | | |

**Table 13 Percentage of subjects with antibody concentrations ≥ 0.2 µg/mL, OPA ≥ 1:8 and GMCs/GMTs against 19A pneumococci one month following primary vaccination with 1µg 19F-PD, 3µg 19F-DT or Prevnar (2µg 19F-CRM) (Total cohort)**

| **Group** | **11Pn-PD&Di-001** | | | | | |
|---|---|---|---|---|---|---|
| | **22F-ELISA assay** | | | **OPA assay** | | |
| | **N** | **% ≥ 0.20 µg/mL** | **GMC (µg/mL)** | **N** | **% ≥ 1:8** | **GMT** |
| | | (95% Cl) | (95% Cl) | | (95% Cl) | (95% Cl) |
| 11Pn-PD | 45 | **28.9** | **0.09** | 52 | **7.7** | **5.2** |
| | | (16.4-44.3) | (0.07-0.11) | | (2.1-18.5) | (4.0-6.8) |
| 19F-DT Form 2^{Γ} | 51 | **29.4** | **0.11** | 59 | **27.1** | **12.4** |
| | | (17.5-43.8) | (0.08-0.16) | | (16.4-40.3) | (7.6-20.3) |
| *Prevnar* | 55 | **18.2** | **0.10** | 61 | **3.3** | **4.6** |
| | | (9.1-30.9) | (0.08-0.12) | | (0.4-11.3) | (3.8-5.6) |

| | | | | | | |
|---|---|---|---|---|---|---|
| **^{Γ} The composition of the different formulations is provided in Table 4** | | | | | | |

### Example 5:

### Inhibition of anti-PS18C antibodies by polysaccharides with different O-acetylation levels

### 1. Introduction

Pneumococcal capsular type specific antibody (anti-PnPS Ab) inhibition assays were developed to compare the epitopes of pneumococcal capsular polysaccharides (PS) in its native form or after its processing for conjugation. In addition to the characterization of the inventor's conjugates, the tests were also used to investigate the level of O-acetylation present in the Prevnar 18C(-CRM) conjugate.

The objective of the experiments reported here was to compare native polysaccharide 18C lots and other 18C PS with different levels O-acetyl groups present on the PS in those assays. Anti-SP1008 (Example 3), 18C-TT (Example 2), 18C-DT and Prevnar sera have been used.

### 2. Materials and Methods

### Polysaccharides

- PS18C ox 007 : 17% O-Acetyl content (by NMR)
   - Produced following the method described in WO 96/05859 example 4 (hydrolysis by acetic acid) followed by oxidation with periodate
- PS18C ox 011: 79% O-Acetyl content (by NMR)
   - Produced from a micro-fluidized PS followed by mild hydrolysis with hydrogen peroxide and oxidation with periodate

### Sera

Type specific antibodies were obtained by pooling sera of infants from study 11 PnPD&Di007 immunized either with SP1008 (11Pn-PD) or with Prevnar. Mice and guinea pig pooled sera were obtained from animals immunized with SP1008, Prevnar or experimental formulations containing PS18-DT_{AH} or PS18-TT_{AH} (AH = with ADH linker) conjugates.

### Methods

### ELISA:

- Microplates were coated with native 18C PS.
- Anti-PnPS 18C Abs were inhibited by pre-incubation with serial 2-fold dilutions of PS 79% O-acetylated or de-O-acetylated and then added to coated microplates.
- Plates were then processed as per usual anti-PS Elisa assay procedure.
- Percentage of inhibition of the anti-PnPS sera (inhibited vs non-inhibited) were calculated and plotted versus inhibitor concentration.

### Opsonophagocytosis

- Anti-PnPS 18C Abs diluted to obtain 95 to 100% killing against the *S*. *pneumoniae* 18C strain in a classical opsonophagocytosis assay were inhibited by pre-incubation with serial 2-fold dilutions of PS 79% O-acetylated or de-O-acetylated and introduced in the opsonophagocytosis reaction mix.
- Subsequently the oponophagocytosis test against *S*. *pneumoniae* was realized as per usual.
- Percentage of inhibition of the anti-PnPS sera (inhibited vs non-inhibited) were calculated and plotted versus inhibitor concentration.

### 3. Results

In order to determine the importance of the O-acetylation level on the 18C PS, inhibition ELISA and opsonophagocytosis experiments were realized. Poorly O-acetylated 18CPS were obtained by acetic acid hydrolysis and periodate oxidation. The two PS tested in inhibition ELISA and OPA were PS18C007 = 17% Oacetyl and PS18C011 = 79% OAcetyl.

### 3.1 Mice ELISA and opsonophagocytosis experiments

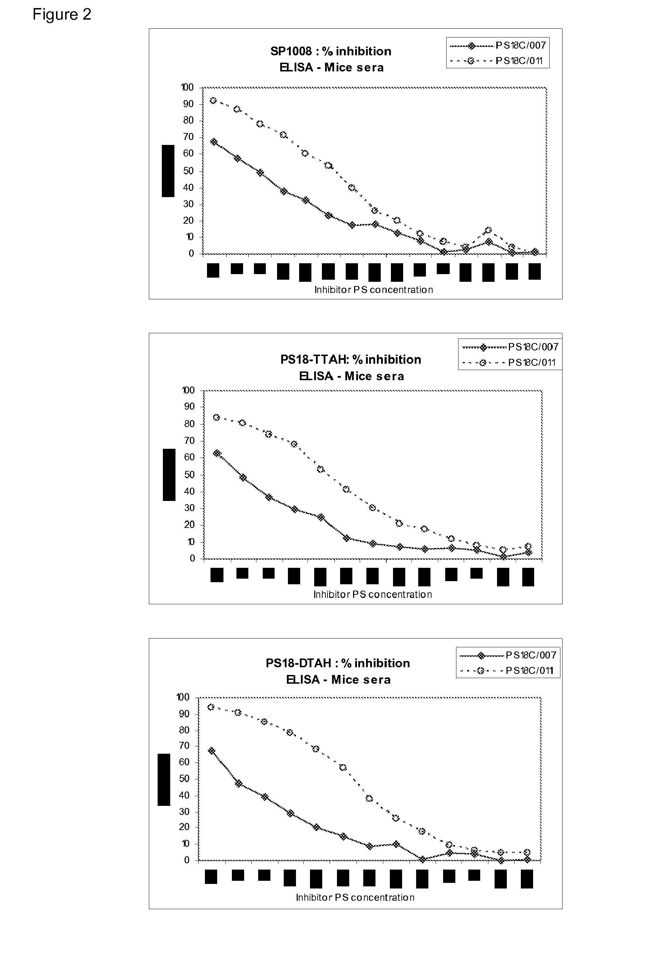

- ELISA inhibition of pooled sera from mice immunized with SP1008 or Prevnar (Figure 1).
- ELISA inhibition of pooled sera from mice immunized with SP1008 or experimental formulations containing PS18-DT_{AH} or PS18-TT_{AH} conjugates (Figure 2).

The ELISA uses an O-acetylated "native" PS as coating antigen and increasing concentrations of PS OAc (011) or de-OAc PS (007) as inhibitors. It is striking that anti-Prevnar Ab's are equally inhibited by PS18C 79% O-acetylated or de-OAc whilst on the contrary anti- 18C-PD, 18C-TT_{AH} and 18C-DT_{AH} sera are more easily inhibited by PS18C 79% O-acetylated than de-OAc.

This suggests that mice immunized with PS18C-PD conjugates produce antibodies reacting to the O-acetylated and to the backbone epitopes of the PS. This finding makes sense given that the 18C PS of this conjugate is highly O-acetylated and the predisposition of the Balb\c mice B-cell repertoire to react with O-acetylated epitopes.

Despite this preference, mice immunized with Prevnar almost do not produce antibodies reacting with the O-acetylated epitope (79% and de-O-acetylated PS inhibit similarly the binding of anti-Prevnar antibodies to the native PS) which strongly suggest that Prevnar PS18C(-CRM) is non or very poorly O-acetylated.

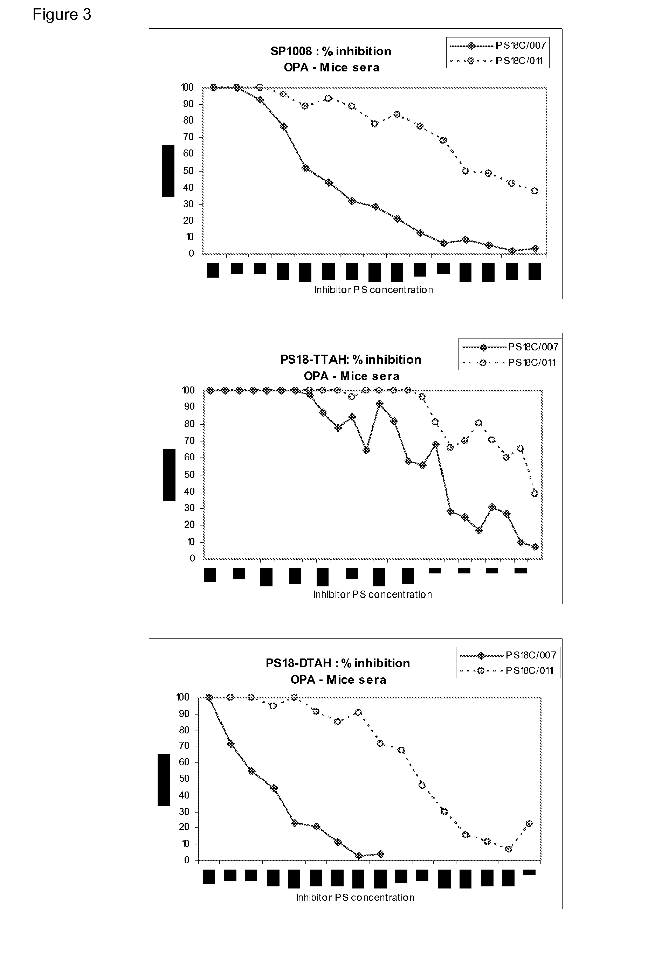

-Opsonophagocytosis inhibition of pooled sera from mice immunized with SP1008 or experimental formulations containing PS18-DT_{AH} or PS18-TT_{AH} conjugates (Figure 3).

In OPA assay, higher inhibition of killing mediated by the antibodies was also observed with the highly O-acetylated polysaccharide. Nevertheless, the O-acetyl content of the strain used in opsonophagocytosis assay is not known.

### 3.2 Guinea pig ELISA and opsonophagocytosis experiments

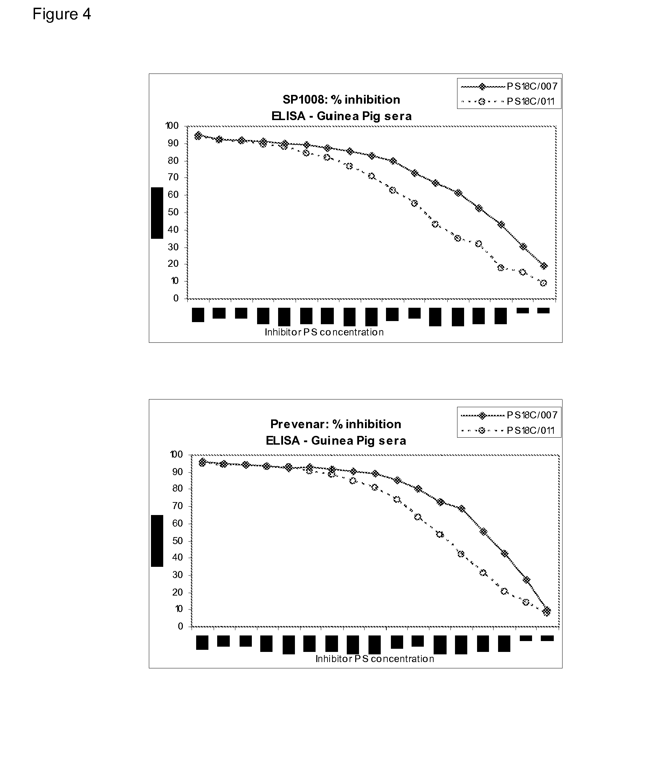

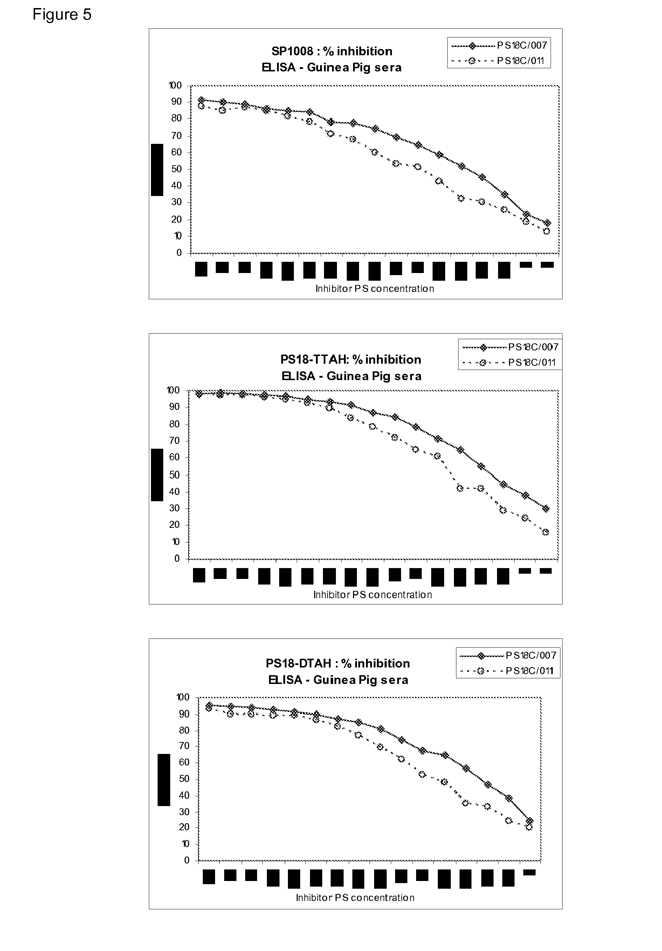

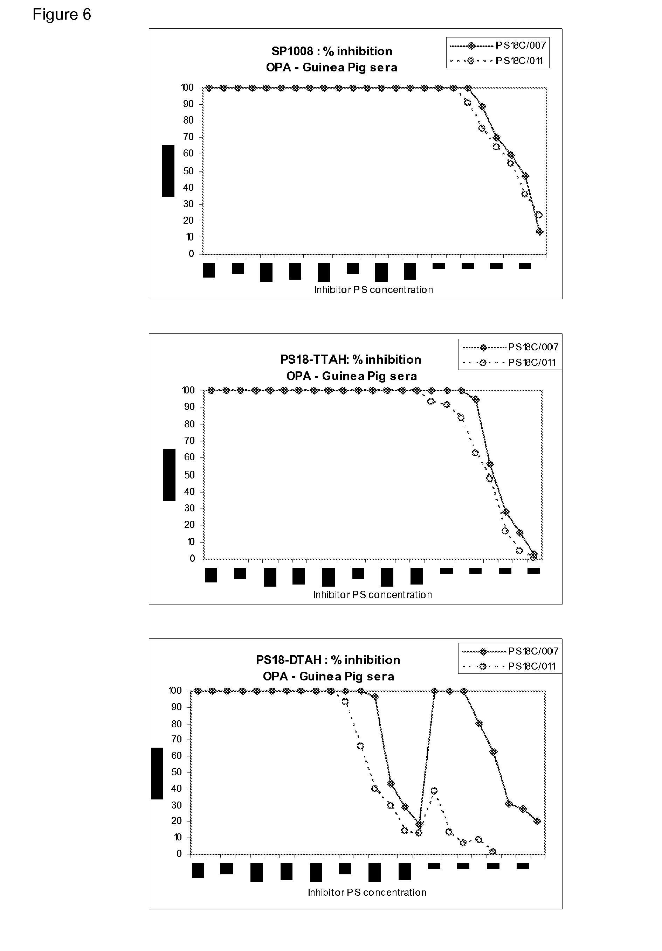

- ELISA inhibition of pooled sera from guinea pig immunized with SP1008 or Prevnar (Figure 4).
- ELISA inhibition of pooled sera from guinea pig immunized with SP1008 or experimental formulations containing PS18-DT_{AH} or PS18-TT_{AH} conjugates (Figure 5).
- Opsonophagocytosis inhibition of pooled sera from guinea pig immunized with SP1008 or experimental formulations containing PS18-DT_{AH} or PS18-TT_{AH} conjugates (Figure 6).

In guinea pigs, no clear differences were observed in inhibition ELISA or in OPA suggesting that guinea pigs produce antibodies mostly directed against the backbone epitopes of the polysaccharide 18C and are not influenced by the O-acetyl content of this antigen.

### 3.3 Human sera experiments

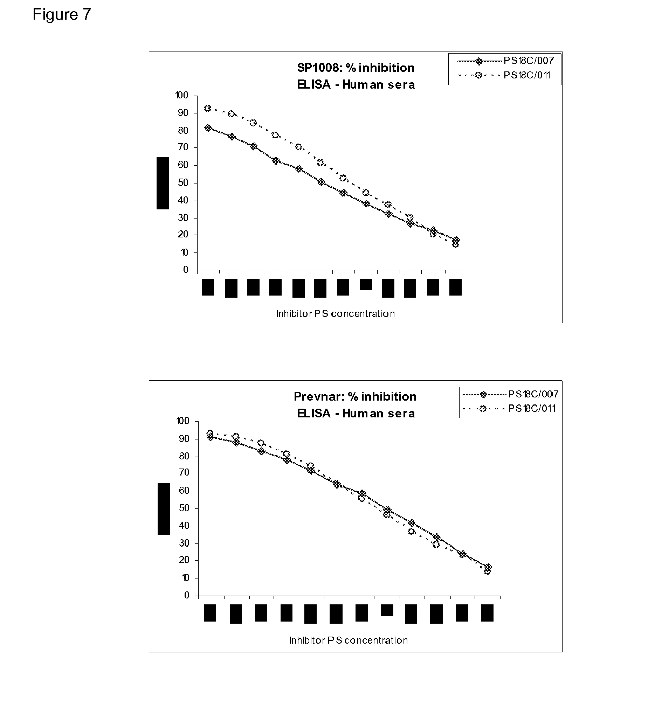

Inhibition of the pooled sera from 11 PnPD&Di007 infants immunized with SP1008 or Prevnar (Figure 7).

As observed with mice antisera, a stronger inhibition of infants anti-18C-PD antibodies is observed with the 79% O-acetylated PS. The 18C PS used to make the SP1008 conjugate is highly O-acetylated (over 90%).

This indicates that infants have produced antibodies to both the backbone but also against the O-acetylated epitopes of the 18C PS. The observation that the anti-prevnar (18C-CRM) sera are similarly inhibited by the 79% O-acetylated and the de-O-acetylated PS indicates that the anti-Prevnar antibodies are almost only reacting with the backbone PS epitopes. This together with the mice sera observation indicate that the 18C PS that is in the Prevnar 18C-CRM conjugate is non or very poorly O-acetylated.

### 4. Discussion

- PS 18C with 79% O-acetyl showed better capacity to neutralize mice and infant antibodies to 11Pn-PD (SP1008) antibodies than the PS 18C with only 17% O-acetyl in both ELISA and Opsonophagocytosis assays.
- Both PS 18C with 79% O-acetyl and with 17% O-acetyl showed same capacity to neutralize Prevnar sera.
- This suggests a very low or a non- O-acetylation of the PS18C of the Prevnar vaccine compared to GSK 18C conjugate vaccines.
- Focusing the immune response against the backbone epitopes may be beneficial in eliciting an antibody response against both 18C strains that are highly O-acetylated and those that are poorly O-acetylated.

## Claims

1. A *Streptococcus pneumoniae* immunogenic composition comprising 10 or more, 11 or more, or 13 or more capsular saccharides from different *S. pneumoniae* serotypes which are conjugated to a carrier protein, wherein the composition comprises conjugated capsular saccharide 18C which is less than 80, 70, 60, 50, 40, 30, 20, 15 or 10% O-Acetylated further comprising capsular saccharides from serotypes 1, 4, 5, 6B, 7F, 9V, 14, 18C, 19F and 23F as conjugated capsular saccharides.

2. An immunogenic composition according to claim 1 wherein the capsular saccharide 18C is conjugated to TT.

3. An immunogenic composition according to any preceding claim, wherein 18C capsular saccharide is directly conjugated to the carrier protein, or wherein 18C capsular saccharide is conjugated to the carrier protein via a linker.

4. The immunogenic composition of any preceding claim wherein the ratio of carrier protein to 18C saccharide is between 1:5 and 5:1, 1:1 and 4:1, 1.5:1 and 3:1, or 2:1 and 2.5:1 (w/w).

5. The immunogenic composition of any preceding claim wherein the dose of the 18C saccharide conjugate is between 1 and 10µg, 1 and 5µg, or 1 and 3µg of saccharide.

6. The *Streptococcus pneumoniae* immunogenic composition of any preceding claim, wherein the vaccine comprises serotype 19F capsular saccharide conjugated to diphtheria toxoid (DT) or CRM197.

7. The immunogenic composition of any preceding claim wherein the 19F capsular saccharide is more than 50, 60, 70, 80, or 90% N-Acetylated.

8. The immunogenic composition of any preceding claim, wherein capsular saccharide from serotype 4 is more than 50, 60, 70, 80, 90% N-Acetylated.

9. The immunogenic composition of any preceding claim wherein capsular saccharide from serotype 9V is more than 50, 60, 70, 80, 90% N-Acetylated and/or more than 50, 60, 70, 80, 90 % O-Acetylated.

10. The immunogenic composition of any preceding claim, wherein capsular saccharide from serotype 14 is more than 50, 60, 70, 80, 90 % N-Acetylated.

11. The immunogenic composition of any preceding claim, wherein capsular saccharide from serotype 1 is more than 50, 60, 70, 80, 90% N-Acetylated and/or wherein capsular saccharide from serotype 1 is more than 50, 60, 70, 80, 90% O-Acetylated.

12. The immunogenic composition of any preceding claim wherein capsular saccharide from serotype 5 is more than 50, 60, 70, 80, 90 % N-Acetylated.

13. The immunogenic composition of any preceding claim wherein capsular saccharide from serotype 7F is more than 50, 60, 70, 80, 90 % N-Acetylated and/or more than 50, 60, 70, 80, 90 % O-Acetylated.

14. The immunogenic composition of any preceding claim wherein the composition also comprises one or more conjugated capsular saccharides from serotypes 3, 6A, 19A and 22F.

15. The immunogenic composition of any preceding claim wherein the M_{W} of the saccharides is above 50x10³, optionally less than 1 x 10⁶.

16. The immunogenic composition of any preceding claim wherein the dose of the capsular saccharide conjugates is between 1 and 10µg, 1 and 5µg or 1 and 3µg of saccharide per conjugate.

17. A vaccine comprising the immunogenic composition of any one of claims 1-16 further comprising an adjuvant, wherein the adjuvant is an aluminium salt such as aluminium hydroxide gel (alum) or aluminium phosphate.
